(19) Europäisches Patentamt
European Patent Office
Office européen des brevets

(11) EP 2 196 187 A1

(12) # DEMANDE DE BREVET EUROPEEN

(43) Date de publication:
**16.06.2010 Bulletin 2010/24**

(51) Int Cl.:
***A61K 8/44*** *(2006.01)* ***A61Q 19/00*** *(2006.01)*

(21) Numéro de dépôt: **09178776.2**

(22) Date de dépôt: **10.12.2009**

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **15.12.2008 FR 0858566**
**15.12.2008 FR 0858568**
**15.12.2008 FR 0858590**
**15.12.2008 FR 0858599**
**22.12.2008 US 193761 P**
**22.12.2008 US 193762 P**
**22.12.2008 US 193763 P**
**22.12.2008 US 193764 P**

(71) Demandeur: **L'Oréal**
**75008 Paris (FR)**

(72) Inventeurs:
- **Kermorvan, Cécile**
**94100 Saint Maur des Fosses (FR)**
- **Chevalier, Véronique**
**94440 Villecresnes (FR)**

(74) Mandataire: **Le Coupanec, Pascale A.M.P.**
**Nony & Associés**
**3, rue de Penthièvre**
**75008 Paris (FR)**

(54) **Composition cosmétique et/ou dermatologique à base d'ester(s) de l'acide N,N'-diarylméthylène éthylènediaminediacétique**

(57) La présente invention concerne une composition cosmétique et/ou dermatologique comprenant, dans un milieu physiologiquement acceptable contenant au moins une huile :
(a) au moins un composé de formule générale (Ia) :

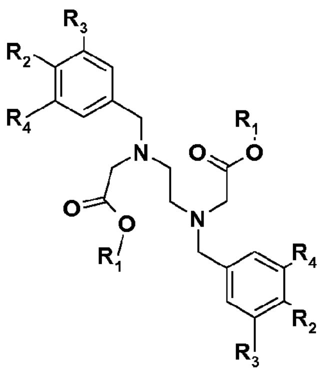

(Ia)

et
(b) une quantité efficace d'au moins un solvant choisi parmi :
(i) l'isononanoate d'isononyle ;
(ii) le diméthyl isosorbide ;
(iii) les esters d'acide aminé de formule (II) :

$$R'_1(CO)N(R'_2)CH(R'_3)(CH_2)_n(CO)OR'_4 \quad (II)$$

(iv) et l'un de leurs mélanges.

EP 2 196 187 A1

## Description

La présente invention concerne des compositions cosmétiques et/ou dermatologiques comprenant au moins un ester de l'acide N,N'-diarylméthylène éthylènediaminediacétique et au moins un solvant particulier.

Les compositions selon l'invention sont en particulier destinées au soin et/ou au maquillage des matières kératiniques, et notamment de la peau.

Des esters de l'acide N,N'-diarylméthylène éthylènediaminediacétique sont décrits dans le document WO 94/11338 comme chélateurs du fer et piégeurs contre la formation de radicaux libres hydroxyle, les rendant intéressants pour protéger les matières kératiniques, et notamment la peau, contre le stress oxydant provoqué notamment par les rayons ultraviolets. Ces dérivés sont donc utiles dans la prévention du photo-vieillissement cutané.

Ces composés présentent toutefois la particularité d'être solides à température ambiante et de n'être que peu ou pas solubles dans les matières premières liquides utilisées de façon usuelle en cosmétique et/ou en dermatologie. En particulier, ces composés ne sont pas solubles dans l'eau, et sont peu solubles dans les polyols tels que la glycérine, le propylène glycol, et également peu solubles dans les huiles apolaires comme l'isododécane, le polyisobutène hydrogéné (Parleam de chez NOF Corporation), le cyclopentasiloxane.

Or, il nécessaire que ces composés soient formulés sous une forme solubilisée afin de tirer au mieux profit de leur activité et il est également préférable que leur solubilisation soit maintenue au cours du temps pour éviter tout phénomène de recristallisation lors du stockage des compositions comprenant de tels composés.

La présente invention a précisément pour objet de proposer une nouvelle formulation galénique d'esters de l'acide N,N'-diarylméthylène éthylènediaminediacétique permettant de s'affranchir des inconvénients précités, et donc d'incorporer ces composés sous une forme solubilisée et durable dans le temps.

Les inventeurs ont en effet découvert, de façon inattendue, que l'association de certains esters de l'acide N, N'-diarylméthylène éthylènediaminediacétique avec au moins un solvant particulier permet de solubiliser ces composés tout en évitant leur recristallisation, notamment après un stockage de 2 mois à la température ambiante (25 °C).

L'invention concerne ainsi, selon un de ses aspects, une composition cosmétique et/ou dermatologique comprenant, dans un milieu physiologiquement acceptable contenant au moins une huile :

(a) au moins un composé de formule générale (I) :

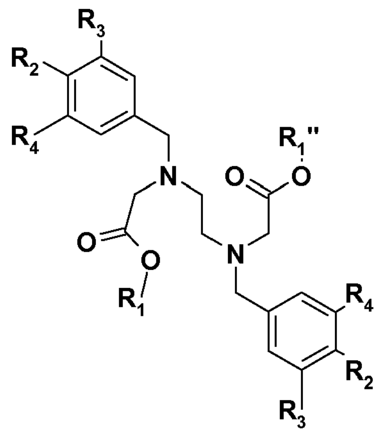

(I)

dans laquelle :

- chaque groupement $R_1$ et $R''_1$ représente indépendamment un atome d'hydrogène ou un radical alkyle saturé linéaire comprenant de 1 à 6 atomes de carbone ou un radical alkyle saturé ramifié comprenant de 3 à 6 atomes de carbone, sous réserve que les deux groupements $R_1$ et $R''_1$ ne représentent pas simultanément un atome d'hydrogène,
- $R_2$, $R_3$ et $R_4$ représentent, indépendamment les uns des autres, un atome d'hydrogène ou un radical $-OR_5$, et
- $R_5$ représente un atome d'hydrogène ou un radical alkyle saturé linéaire comprenant de 1 à 5 atomes de carbone ou un radical alkyle saturé ramifié comprenant de 3 à 5 atomes de carbone,

ou leurs sels, et

(b) une quantité efficace d'au moins un solvant choisi parmi :

(i) l'isononanoate d'isononyle ;
(ii) le diméthyl isosorbide ;
(iii) les esters d'acide aminé de formule (II) :

$$R'_1(CO)N(R'_2)CH(R'_3)(CH_2)"(CO)OR'_4 \quad (II)$$

dans laquelle :

- n est un entier égal à 0, 1 ou 2,
- $R'_1$ représente un radical alkyle ou alcényle en $C_5$ à $C_{21}$, linéaire ou ramifié,
- $R'_2$ représente un atome d'hydrogène ou un groupe alkyle en $C_1$ à $C_3$,
- $R'_3$ représente un radical choisi dans le groupe formé par un atome d'hydrogène, un groupe méthyle, un groupe éthyle, un radical alkyle linéaire ou ramifié en $C_3$ ou $C_4$, et
- $R'_4$ représente un radical alkyle en $C_1$ à $C_{10}$ linéaire ou ramifié, ou un radical alcényle en $C_2$ à $C_{10}$ linéaire ou ramifié, ou un reste stérol ; et

(iv) l'un de leurs mélanges.

Comme précisé ci-après, ledit composé de formule générale (I) est avantageusement présent, dans les compositions conformes à l'invention, sous une forme solubilisée.

Les compositions conformes à l'invention sont avantageusement mises en oeuvre pour le soin et/ou le maquillage des matières kératiniques, et notamment de la peau.

L'association d'un ester de l'acide N,N'-diarylméthylène éthylènediaminediacétique conforme à l'invention avec un tel solvant permet d'obtenir des compositions cosmétiques ou dermatologiques remarquablement efficaces pour les applications mentionnées précédemment.

Les compositions conformes à l'invention peuvent notamment être destinées à traiter et/ou protéger les matières kératiniques d'êtres humains, en particulier la peau, contre le vieillissement engendré notamment par l'exposition au soleil (rayons ultraviolets).

Ainsi, selon un autre de ses aspects, la présente invention concerne un procédé de traitement non thérapeutique de soin et/ou de maquillage des matières kératiniques, notamment de la peau, comprenant au moins l'étape d'appliquer sur lesdites matières kératiniques, et notamment sur la peau, au moins une composition telle que définie précédemment.

## ESTER DE L'ACIDE N,N'-DIARYLMETHYLENE ETHYLENE DIAMINEDIACETIOUE

Les esters de l'acide N,N'-diarylméthylène éthylènediaminediacétique considérés selon l'invention sont des composés de formule générale (I) :

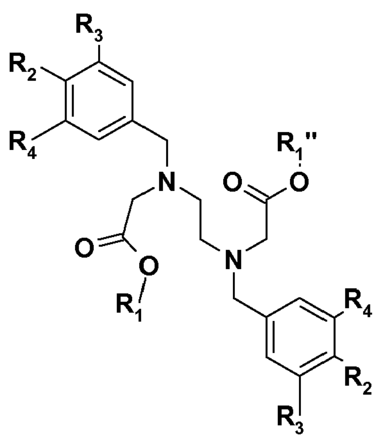

(I)

dans laquelle :

- chaque groupement $R_1$ et $R"_1$ représente indépendamment un atome d'hydrogène ou un radical alkyle saturé

linéaire comprenant de 1 à 6 atomes de carbone ou un radical alkyle saturé ramifié comprenant de 3 à 6 atomes de carbone, sous réserve que les deux groupements $R_1$ et $R''_1$ ne représentent pas simultanément un atome d'hydrogène,

- $R_2$, $R_3$ et $R_4$ représentent, indépendamment les uns des autres, un atome d'hydrogène ou un radical $-OR_5$, et
- $R_5$ représente un atome d'hydrogène ou un radical alkyle saturé linéaire comprenant de 1 à 5 atomes de carbone ou un radical alkyle saturé ramifié comprenant de 3 à 5 atomes de carbone.

Ces composés peuvent être obtenus par toute méthode connue de l'homme du métier et, par exemple, suivant les procédés de préparation décrits dans le document WO 94/11338.

Selon un premier mode de réalisation, il s'agit plus particulièrement d'esters de l'acide N,N'-diarylméthylène éthylènediaminediacétique de formule générale (Ia) :

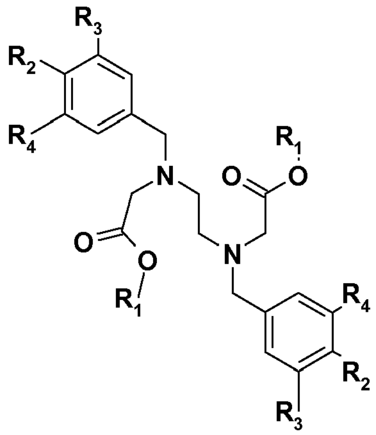

(Ia)

dans laquelle :

- $R_1$ représente un radical alkyle saturé linéaire comprenant de 1 à 6 atomes de carbone ou un radical alkyle saturé ramifié comprenant de 3 à 6 atomes de carbone,
- $R_2$, $R_3$ et $R_4$ représentent, indépendamment les uns des autres, un atome d'hydrogène ou un radical $-OR_5$, et
- $R_5$ représente un atome d'hydrogène ou un radical alkyle saturé linéaire comprenant de 1 à 5 atomes de carbone ou un radical alkyle saturé ramifié comprenant de 3 à 5 atomes de carbone.

Selon un mode de réalisation, $R_2$, $R_3$ et $R_4$ représentent un atome d'hydrogène.

Selon un autre mode de réalisation, $R_1$ désigne un radical isopropyle.

Il peut notamment s'agir du composé de formule générale (Ia) dans laquelle $R_2$, $R_3$ et $R_4$ représentent un atome d'hydrogène et $R_1$ désigne un radical isopropyle, c'est-à-dire de l'ester diisopropylique de l'acide N,N'-bis-(benzyl) éthylènediamine-N-N'-diacétique de formule :

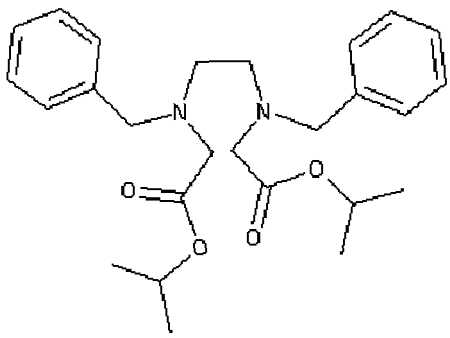

Ce composé est plus particulièrement décrit dans l'exemple 16 du document WO 94/11338.

Selon un deuxième mode de réalisation, il peut également s'agir d'esters de l'acide N,N'-diarylméthylène éthylènediaminediacétique de formule générale (Ib) :

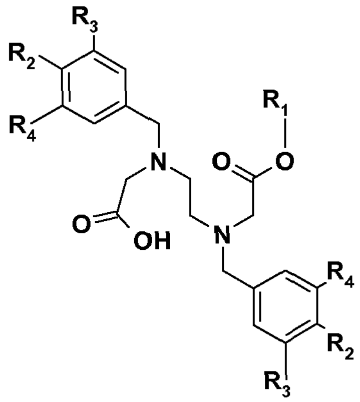

(Ib)

dans laquelle :

- $R_1$ représente un radical alkyle saturé linéaire comprenant de 1 à 6 atomes de carbone ou un radical alkyle saturé ramifié comprenant de 3 à 6 atomes de carbone,
- $R_2$, $R_3$ et $R_4$ représentent, indépendamment les uns des autres, un atome d'hydrogène ou un radical $-OR_5$, et
- $R_5$ représente un atome d'hydrogène ou un radical alkyle saturé linéaire comprenant de 1 à 5 atomes de carbone ou un radical alkyle saturé ramifié comprenant de 3 à 5 atomes de carbone.

Les composés de formule (Ib) sont des composés nouveaux non décrits dans l'art antérieur. L'invention concerne également de tels composés.

Comme composé de formule (Ib), on peut citer le monoester isopropylique de l'acide N,N'-bis-(benzyl)éthylènediamine-N-N'-diacétique.

Les composés de formule (Ib) peuvent être obtenus selon le schéma 1 de synthèse en faisant réagir une éthylènediamine dibenzylée (A) avec un équivalent molaire de bromoacétate de tertio-butyle en présence d'une base telle que par exemple le carbonate de potassium dans un solvant polaire tel que par exemple le diméthylformamide, puis en faisant réagir un équivalent molaire du bromoacétate dérivé de l'alcool $R_1OH$. Le diester intermédiaire (B) obtenu est ensuite hydrolysé sélectivement en sa partie tertiobutylique en milieu acide (par exemple, acide chlorhydrique ou trifluoroacétique) pour conduire à l'hémiester de formule (Ib).

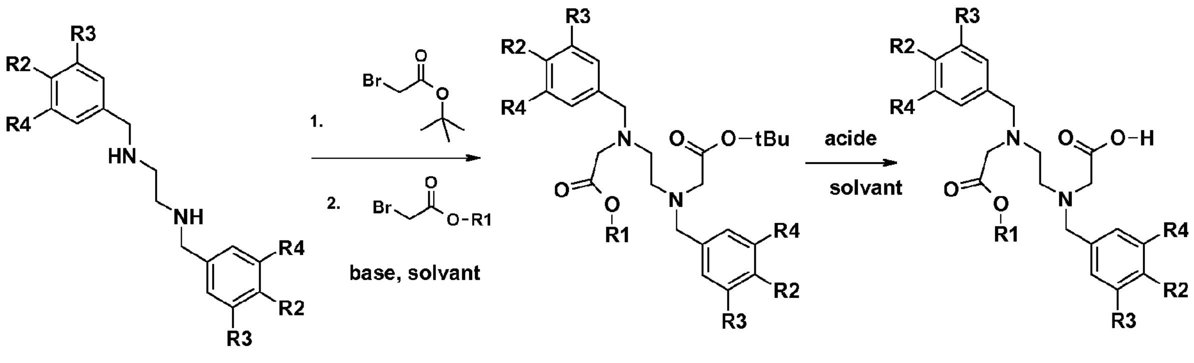


(A) (B) (Ib)

Schéma I

Les sels des composés (I), (Ia) et (Ib) décrits précédemment comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique,

l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide trifluoroacétique, l'acide téréphtalique, l'acide citrique et l'acide tartrique.

Les sels préférés sont ceux obtenus à partir des acides chlorhydrique, sulfurique, acétique, trifluoroacétique, tartrique, citrique.

Les compositions conformes à l'invention peuvent comprendre de 0,01 à 5 % en poids, de préférence de 0,1 à 2,5 % en poids, notamment de 0,5 à 1 % en poids de composé de formule générale (I), (Ia) ou (Ib), en particulier de formule (Ia), par rapport au poids total de ladite composition.

## SOLVANT

Les compositions conformes à l'invention comprennent une quantité efficace d'au moins un solvant choisi parmi :

(i) l'isononanoate d'isononyle ;
(ii) le diméthyl isosorbide ;
(iii) les esters d'acide aminé de formule (II) :

$$R'_1(CO)N(R'_2)CH(R'_3)(CH_2)''(CO)OR'_4 \qquad (II)$$

dans laquelle :

- n est un entier égal à 0, 1 ou 2,
- $R'_1$ représente un radical alkyle ou alcényle en $C_5$ à $C_{21}$, linéaire ou ramifié,
- $R'_2$ représente un atome d'hydrogène ou un groupe alkyle en $C_1$ à $C_3$,
- $R'_3$ représente un radical choisi dans le groupe formé par un atome d'hydrogène, un groupe méthyle, un groupe éthyle, un radical alkyle linéaire ou ramifié en $C_3$ ou $C_4$, et
- $R'_4$ représente un radical alkyle en $C_1$ à $C_{10}$ linéaire ou ramifié, ou un radical alcényle en $C_2$ à $C_{10}$ linéaire ou ramifié, ou un reste stérol ; et

(iv) l'un de leurs mélanges.

Selon un premier mode de réalisation de l'invention, le solvant est l'isononanoate d'isononyle.

Selon un deuxième mode de réalisation de l'invention, le solvant est le diméthyl isosorbide.

Selon un troisième mode de réalisation, le solvant est un ester d'acide aminé de formule (II) telle qu'indiquée précédemment.

Les esters d'acide aminé convenant à l'invention, ainsi que leur procédé de synthèse sont notamment décrits dans les demandes de brevet EP 1 044 676 et EP 0 928 608 de la société AJINOMOTO Co.

Dans les esters d'acide aminé de formule (II), le groupement $R'_1(CO)$- est de préférence un groupement acyle d'un acide choisi de préférence dans le groupe formé par l'acide caprique, l'acide laurique, l'acide myristique, l'acide palmitique, l'acide stéarique, l'acide béhénique, l'acide linoléique, l'acide linolénique, l'acide oléique, l'acide isostéarique, l'acide 2-éthylhexanoïque, les acides gras d'huile de coco, les acides gras d'huile de palmiste. Ces acides gras peuvent en outre présenter un groupe hydroxyle. De manière encore plus préférée, il s'agira de l'acide laurique.

La partie $-N(R'_2)CH(R'_3)(CH_2)_n(CO)$- de l'ester d'aminoacide est de préférence choisie parmi les aminoacides suivants : glycine, alanine, valine, leucine, isoleucine, sérine, thréonine, proline, hydroxyproline, β-alanine, acide aminobutyrique, acide aminoca-proique, sarcosine, ou N-méthyl-β-alanine.

De manière encore plus préférée, il s'agira de la sarcosine.

La partie des esters aminoacides correspondant au groupe $OR'_4$ peut être obtenue à partir des alcools choisis dans le groupe formé par le méthanol, éthanol, propanol, isopropanol, butanol, tert-butanol, isobutanol, 3-méthyl-1-butanol, 2-méthyl-1-butanol, huile de fusel, pentanol, héxanol, cyclohéxanol, octanol, 2-éthylhéxanol, décanol, alcool laurylique, alcool myristique, alcool cétylique, alcool cétostéarylique, alcool stéarylique, alcool oléique, alcool béhénylique, alcool de jojoba, alcool 2-héxadécylique, alcool 2-octyldodécanol et alcool isostéarylique.

Ces esters d'acide aminé peuvent en particulier être obtenus à partir de sources naturelles en acides aminés. Dans ce cas, les acides aminés proviennent d'hydrolyse de protéines naturelles végétales (avoine, blé, soja, palme, coco) et conduisent alors nécessairement à des mélanges d'acides aminés qui devront ensuite être estérifiés puis N-acylés. La préparation de tels acides aminés est plus particulièrement décrite dans la demande de brevet FR 2 796 550

qui est incorporée ici par référence.

L'ester d'acide aminé plus particulièrement préféré pour son utilisation dans la présente invention est le N-lauroylsarcosinate d'isopropyle de formule $CH_3-(CH_2)_{10}CON(CH_3)-CH_2-COO-CH(CH_3)_2$.

A titre de N-lauroylsarcosinate d'isopropyle, on peut, par exemple, citer le produit commercialisé par la société AJINOMOTO sous la référence ELDEW SL-205®.

Dans le cadre de la présente invention, il est entendu par quantité efficace de solvant ou mélange de solvants conforme à l'invention une quantité suffisante de ce solvant ou mélange de solvants pour solubiliser l'ester de l'acide N,N'-diarylméthylène éthylènediaminediacétique de formule (I), (Ia) ou (Ib), et donc prévenir tout phénomène de recristallisation, notamment au cours du stockage.

Pour des raisons évidentes, la quantité de solvant conforme à l'invention suffisante pour solubiliser l'ester de l'acide N,N'-diarylméthylène éthylènediaminediacétique est susceptible de varier dans une large mesure en fonction notamment de la nature chimique et/ou de la quantité dudit ester de l'acide N,N'-diarylméthylène éthylènediaminediacétique à solubiliser et de la nature du solvant ou du mélange de solvants utilisés. L'ajustement de la quantité de solvant conforme à l'invention fait partie des compétences de l'homme du métier.

Il est clair que dans les modes de réalisation dans lesquels ce solvant est figuré par l'un des trois solvants précités ou un de leur mélange, la présence conjointe d'un autre solvant est envisageable, sous réserve bien évidemment que ce composé annexe ne porte pas préjudice à la solubilité assurée par le solvant requis selon l'invention.

A titre illustratif, le solvant selon l'invention peut être présent en une quantité allant de 0,05 à 25 % en poids, plus préférentiellement de 0,5 à 12,5 % en poids, et plus particulièrement de 2,5 à 5 % en poids par rapport au poids total de la composition.

Selon un mode de réalisation, le solvant conforme à l'invention et le composé de formule générale (I), (Ia) ou (Ib) peuvent être présents en un rapport massique [solvant/composé de formule générale (I), (Ia) ou (Ib)] au moins égal à 1,5, notamment allant de 1,5 à 15, et de préférence allant de 1,5 à 10.

Selon un mode de réalisation, le solvant conforme à l'invention et le composé de formule générale (Ia) peuvent être présents en un rapport massique [solvant/composé de formule générale (Ia)] au moins égal à 1,5, notamment allant de 1,5 à 15, et de préférence allant de 1,5 à 10.

Lorsque le solvant est l'isononanoate d'isononyle, le rapport massique [isononanoate d'isononyle/composé de formule générale (I), (Ia) ou (Ib)] peut être notamment au moins égal à 5,25, par exemple allant de 5,25 à 8, et de préférence allant de 5,25 à 6,5.

En particulier, le rapport massique [isononanoate d'isononyle/composé de formule générale (Ia)] peut être notamment au moins égal à 5,25, par exemple allant de 5,25 à 8, et de préférence allant de 5,25 à 6,5.

Lorsque le solvant est le diméthyl isosorbide, le rapport massique [diméthyl isosorbide/composé de formule générale (I), (Ia) ou (Ib)] peut être notamment au moins égal à 1,6, par exemple allant de 1,6 à 10, de préférence allant de 1,6 à 6.

En particulier, le rapport massique [diméthyl isosorbide/composé de formule générale (Ia)] peut être notamment au moins égal à 1,6, par exemple allant de 1,6 à 10, de préférence allant de 1,6 à 6.

Lorsque le solvant est un ester d'acide aminé de formule (II), le rapport massique [ester d'acide aminé de formule (II)/composé de formule générale (I), (Ia) ou (Ib)] peut être notamment au moins égal à 4,5, par exemple allant de 4,5 à 10, de préférence allant de 4,5 à 6.

En particulier, le rapport massique [ester d'acide aminé de formule (II)/composé de formule générale (Ia)] peut être notamment au moins égal à 4,5, par exemple allant de 4,5 à 10, de préférence allant de 4,5 à 6.

En particulier, la rapport massique [N-lauroylsarcosinate d'isopropyle/ester diisopropylique de l'acide N,N'-bis-(benzyl)éthylènediamine-N-N'-diacétique] peut être au moins égal à 4,5, par exemple allant de 4,5 à 10, de préférence allant de 4,5 à 6.

## MILIEU PHYSIOLOGIQUEMENT ACCEPTABLE

Les compositions utilisées selon l'invention contiennent un milieu physiologiquement acceptable, c'est-à-dire compatible avec les tissus cutanés tels que la peau et le cuir chevelu.

Outre les solvants conformes à l'invention mentionnés précédemment, ce milieu physiologiquement acceptable comprend au moins une huile différente des solvants décrits précédemment.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société

Stearineries Dubois ou ceux vendus sous les dénominations « Miglyol 810 », « 812 » et « 818 » par la société Dynamit Nobel, l'huile de jojoba, l'huile de beurre de karité;

- les esters et les éthers de synthèse, notamment les esters d'acides gras, comme les huiles de formules $R_1COOR_2$ et $R_1OR_2$ dans laquelle $R_1$ représente le reste d'un acide gras comportant de 8 à 29 atomes de carbone, et $R_2$ représente une chaîne hydrocarbonée, ramifiée ou non, contenant de 3 à 30 atomes de carbone, comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle ; les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, acide carboxylique et/ou alcool.

La composition selon l'invention peut comprendre des corps solides à la température ambiante (25 °C) comme par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, les cires microcristallines, la cérésine ou l'ozokérite, les cires synthétiques comme les cires de polyéthylène, les cires de Fischer-Tropsch.

Ces corps gras peuvent être choisis de manière variée par l'homme du métier afin de préparer une composition ayant les propriétés, par exemple de consistance ou de texture, souhaitées.

La composition selon l'invention peut comprendre de l'eau et éventuellement un solvant organique physiologiquement acceptable choisi par exemple parmi les alcools inférieurs comportant de 1 à 8 atomes de carbone et en particulier de 1 à 6 atomes de carbone, comme l'éthanol, l'isopropanol, le propanol, le butanol ; les polyéthylène glycols ayant de 6 à 80 unités oxyde d'éthylène et les polyols comme le propylène glycol, l'isoprène glycol, le butylène glycol, la glycérine et le sorbitol.

Avantageusement, les compositions selon l'invention peuvent se présenter sous la forme d'émulsion, notamment d'émulsion huile-dans-eau, d'émulsion eau-dans-huile, d'émulsion triple E/H/E ou H/E/H, de dispersions d'une phase grasse dans une phase aqueuse à l'aide de sphérules, ces sphérules pouvant être des nanoparticules polymériques telles que les nanosphères et les nanocapsules ou des vésicules lipidiques de type ionique et/ou non ionique (liposomes, niosomes, oléosomes). Ces compositions sont préparées selon les méthodes usuelles.

En outre, les compositions selon l'invention peuvent être plus ou moins fluides et avoir l'aspect d'une crème blanche ou colorée, d'une pommade, d'un lait, d'une lotion, d'un sérum, d'une pâte ou d'une mousse. Elles peuvent être éventuellement appliquées sur la peau sous forme d'aérosol. Elles peuvent aussi se présenter sous forme solide, et par exemple sous forme de stick.

Selon un mode particulier de réalisation de l'invention, la composition selon l'invention est une émulsion eau-dans-huile (E/H) ou huile-dans-eau (H/E), de préférence une émulsion huile-dans-eau. La proportion de la phase huileuse de l'émulsion peut aller de 5 à 80 % en poids, et de préférence de 5 à 50 % en poids par rapport au poids total de la composition.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les diméthicone copolyols tels que le mélange de cyclométhicone et de diméthicone copolyol, vendu sous la dénomination « DC 5225 C » par la société Dow Corning, et les alkyl-diméthicone copolyols tels que le Laurylméthicone copolyol vendu sous la dénomination « Dow Corning 5200 Formulation Aid » par la société Dow Corning et le Cetyl dimethicone copolyol vendu sous la dénomination « Abil EM 90® » par la société Goldschmidt.

On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004 et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

De façon connue, la composition cosmétique ou dermatologique de l'invention peut contenir également des adjuvants habituels dans le domaine cosmétique ou dermatologique tels que les gélifiants, les polymères filmogènes, les conservateurs, les parfums, les charges, les filtres UV, les bactéricides, les absorbeurs d'odeur, les matières colorantes, les extraits végétaux, les actifs cosmétiques et dermatologiques, et les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

La composition selon l'invention peut comprendre, en outre, au moins un agent photoprotecteur organique et/ou au moins un agent photoprotecteur inorganique actif dans l'UVA et/ou l'UVB (absorbeurs) pouvant être hydrosolubles ou liposolubles ou bien insolubles dans les solvants cosmétiques couramment utilisés.

De préférence, on utilisera un système filtrant à la fois les radiations UVA et les radiations UVB.

Les filtres solaires sont des molécules qui absorbent les rayonnements UV et permettent ainsi d'éviter que ceux-ci n'arrivent au niveau des cellules de la peau. Ils peuvent absorber soit principalement les UVB, soit principalement les UVA, en fonction de leur nature. Il existe deux grandes catégories de filtres solaires, soit organiques, soit minéraux (oxydes de zinc ou de titane). En les utilisant dans des compositions cosmétiques en association et en quantité suffisante, ils permettent d'arrêter une grande partie du rayonnement UV.

Cependant il est couramment admis que pour être efficaces ces formules doivent être utilisées dans de bonnes conditions d'application (quantité suffisante, renouvellement fréquent, étalement homogène). Ces conditions d'application ne sont pas toujours respectées par l'utilisateur, ce qui augmente le risque qu'une quantité non négligeable de rayonnements UV atteigne les cellules de la peau, et donc engendre les effets biologiques cités plus haut. De plus, pour obtenir une absorption vis-à-vis de l'ensemble des longueurs d'onde du spectre UV solaire UVB + UVA il faut associer plusieurs molécules absorbant dans des domaines de longueurs d'onde complémentaires.

Les filtres organiques complémentaires sont notamment choisis parmi les anthranilates ; les dérivés cinnamiques ; les dérivés salicyliques, les dérivés du camphre ; les dérivés de la benzophénone ; les dérivés de $\beta,\beta$-diphénylacrylate ; les dérivés de triazine ; les dérivés de benzotriazole ; les dérivés de benzalmalonate notamment ceux cités dans le brevet US 5,624,663 ; les dérivés de benzimidazole ; les imidazolines ; les dérivés bis-benzoazolyle tels que décrits dans les brevets EP 0 669 323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB 2303549, DE 197 26 184 et EP 0 893 119 ; les dérivés de benzoxazole tels que décrits dans les demandes de brevet EP 0 832 642, EP 1 027 883, EP 1 300 137 et DE 10 16 2844 ; les polymères filtres et silicones filtres tels que ceux décrits notamment dans la demande WO 93/04665 ; les dimères dérivés d'$\alpha$-alkylstyrène tels que ceux décrits dans la demande de brevet DE 19 85 5649 ; les 4,4-diarylbutadiènes tels que décrits dans les demandes EP 0 967 200, DE 19 74 6654, DE 19 75 5649, EP-A-1 008 586, EP 1 133 980 et EP 1 133 981 ; les dérivés de mérocyanines tels que ceux décrits dans les demandes WO 04/006878, WO 05/058269 et WO 06/032741 et leurs mélanges.

Comme exemples d'agents photoprotecteurs organiques complémentaires, on peut citer ceux désignés ci-dessous sous leur nom INCI :

Dérivés cinnamiques :

Ethylhexyl Methoxycinnamate vendu notamment sous le nom commercial PARSOL MCX par DSM Nutritional Products, Inc,

Isopropyl Methoxycinnamate,
Isoamyl Methoxycinnamate vendu sous le nom commercial NEO HELIOPAN E 1000 par SYMRISE,
DEA Methoxycinnamate,
Diisopropyl Methylcinnamate,
Glyceryl Ethylhexanoate Dimethoxycinnamate,

Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

Dérivés salicyliques :

Homosalate vendu sous le nom « Eusolex HMS » par Rona/EM Industries,
Ethylhexyl Salicylate vendu sous le nom « NEO HELIOPAN OS » par SYMRISE,
Dipropyleneglycol Salicylate vendu sous le nom « DIPSAL » par SCHER,
TEA Salicylate, vendu sous le nom « NEO HELIOPAN TS » par SYMRISE,

Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF,

Dérivés de la benzophénone :

Benzophenone-1 vendu sous le nom commercial « UVINUL 400 » par BASF,
Benzophenone-2 vendu sous le nom commercial « UVINUL D50 » par BASF,
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5,
Benzophenone-6 vendu sous le nom commercial « Helisorb 11 » par Norquay,
Benzophenone-8 vendu sous le nom commercial « Spectra-Sorb UV-24 » par American Cyanamid,
Benzophenone-9 vendu sous le nom commercial« UVINUL DS-49» par BASF,
Benzophenone-12,
2-(4-diethylamino-2-hydroxybenzoyl)-benzoate de n-hexyle vendu sous le nom commercial « UVINUL A+ » par BASF,

Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,

Polyacrylamidomethyl Benzylidene Camphor fabriqué sous le nom « MEXORYL SW » par CHIMEX,

Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial « NEO HELIOPAN AP » par SYMRISE,

Dérivés du phenyl benzotriazole :

Drometrizole Trisiloxane vendu sous le nom « Silatrizole » par RHODIA CHIMIE,
Methylène bis-Benzotriazolyl Tetramethylbutylphénol, vendu sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS,

Dérivés de triazine :

- Bis-Ethylhexyloxyphenol Methoxyphenyl Triazine vendu sous le nom commercial «TINOSORB S » par CIBA GEIGY,
- Ethylhexyl triazone vendu notamment sous le nom commercial « UVINUL T150 » par BASF,
- Diethylhexyl Butamido Triazone vendu sous le nom commercial « UVASORB HEB » par SIGMA 3V,
- la 2,4,6-tris(4'-amino benzalmalonate de dinéopentyle)-s-triazine
- la 2,4,6-tris-(4'-amino benzalmalonate de diisobutyle)-s- triazine,
- la 2,4bis (4'-aminobenzoate de n-butyle)-6-(aminopropyltrisiloxane)-s-triazine,
- la 2,4bis (4'-aminobenzalmalonate de dinéopentyle)-6-(4'-aminobenzoate de n-butyle)-s-triazine,
- les filtres triazines symétriques décrits dans le brevet US 6,225,467, la demande WO 2004/085412 (voir composés 6 et 9) ou le document « Symetrical Triazine Derivatives» IP.COM Journal, IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine et la 2,4,6-tris(terphenyl)-1,3,5-triazine qui est repris dans les demandes de brevet WO 06/035000, WO 06/034982, WO 06/034991, WO 06/035007, WO 2006/034992, WO 2006/034985,

Dérivés anthraniliques :

Menthyl anthranilate vendu sous le nom commercial commercial « NEO HELIOPAN MA » par Haarmann et REIMER,

Dérivés d'imidazolines :

Ethylhexyl Dimethoxybenzylidene Dioxoimidazoline Propionate,

Dérivés du benzalmalonate :

Polyorganosiloxane à fonctions benzalmalonate comme le Polysilicone-15 vendu sous la dénomination commerciale « PARSOL SLX » par DSM Nutritional Products, Inc,

Dérivés de 4,4-diarylbutadiène :

1,1-dicarboxy (2,2'-diméthyl-propyl)-4,4-diphénylbutadiène,

Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V,

Dérivés de mérocyanine :

Octyl-5-N,N-diethylamino-2-phenysulfonyl-2,4-pentadienoate,
et leurs mélanges,

Les filtres organiques préférentiels sont choisis parmi

Ethylhexyl Methoxycinnamate,
Ethylhexyl Salicylate,
Homosalate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle,
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol,
Bis-Ethylhexyloxyphenol Methoxyphenyl Triazine,
Ethylhexyl triazone,
Diethylhexyl Butamido Triazone,
la 2,4,6-tris(4'-amino benzalmalonate de dinéopentyle)-s-triazine,
la 2,4,6-tris-(4'-amino benzalmalonate de diisobutyle)-s- triazine,
la 2,4bis (4'-aminobenzoate de n-butyle)-6-(aminopropyltrisiloxane)-s-triazine,
la 2,4bis (4'-aminobenzalmalonate de dinéopentyle)-6-(4'-aminobenzoate de n-butyle)-s-triazine,
la 2,4,6-tris(biphenyl-4-yl-1,3,5-triazine,
la 2,4,6-tris(terphenyl)-1,3,5-triazine,
Drometrizole Trisiloxane,
Polysilicone-15,
1,1-dicarboxy (2,2'-diméthyl-propyl)-4,4-diphénylbutadiène,
2,4-bis-[5-1(diméthylpropyl)benzoxazol-2-yl-(4-phenyl)-imino]-6-(2-ethylhexyl)-imino-1,3,5-triazine,
Octyl-5-N,N-diethylamino-2-phenysulfonyl-2,4-pentadienoate,
et leurs mélanges.

Les filtres organiques conformes à l'invention peuvent représenter de 0,1 à 30 %, de préférence de 1 à 25 %, du poids total de la composition.

Les filtres UV inorganiques complémentaires utilisés conformément à la présente invention sont des pigments d'oxyde métallique. Plus préférentiellement, les filtres UV inorganiques de l'invention sont des particules d'oxyde métallique ayant une taille moyenne de particule élémentaire inférieure ou égale à 500 nm, plus préférentiellement comprise entre 5 nm et 500 nm, et encore plus préférentiellement comprise entre 10 nm et 100 nm, et préférentiellement entre 15 et 50 nm.

Ils peuvent être notamment choisis parmi les oxydes de titane, de zinc, de fer, de zirconium, de cérium ou leurs

mélanges et plus particulièrement les oxydes de titane.

De tels pigments d'oxydes métalliques, enrobés ou non enrobés sont en particulier décrits dans la demande de brevet EP-A-0 518 773. A titre de pigments commerciaux on peut mentionner les produits vendus les sociétés Kemira, Tayca, Merck et Degussa.

Les pigments d'oxydes métalliques peuvent être enrobés ou non enrobés.

Les pigments enrobés sont des pigments qui ont subi un ou plusieurs traitements de surface de nature chimique, électronique, mécanochimique et/ou mécanique avec des composés tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Les pigments enrobés sont plus particulièrement des oxydes de titane enrobés :

- de silice tels que le produit « SUNVEIL » de la société IKEDA,
- de silice et d'oxyde de fer tels que le produit « SUNVEIL F » de la société IKEDA,
- de silice et d'alumine tels que les produits « MICROTITANIUM DIOXIDE MT 500 SA» et « MICROTITANIUM DIOXIDE MT 100 SA» de la société TAYCA, « TIOVEIL » de la société TIOXIDE,
- d'alumine tels que les produits « TIPAQUE TTO-55 (B) » et « TIPAQUE TTO-55 (A) » de la société ISHIHARA, et « UVT 14/4 » de la société KEMIRA,
- d'alumine et de stéarate d'aluminium tels que les produits « MICROTITANIUM DIOXIDE MT 100 T, MT 100 TX, MT 100 Z, MT-01 » de la société TAYCA, les produits « Solaveil CT-10 W » et « Solaveil CT 100 » de la société UNIQEMA et le produit « Eusolex T-AVO » de la société MERCK,
- de silice, d'alumine et d'acide alginique tel que le produit « MT-100 AQ » de la société TAYCA,
- d'alumine et de laurate d'aluminium tel que le produit « MICROTITANIUM DIOXIDE MT 100 S » de la société TAYCA,
- d'oxyde de fer et de stéarate de fer tels que le produit « MICROTITANIUM DIOXIDE MT 100 F » de la société TAYCA,
- d'oxyde de zinc et de stéarate de zinc tels que le produit « BR 351 » de la société TAYCA,
- de silice et d'alumine et traités par une silicone tels que les produits « MICROTITANIUM DIOXIDE MT 600 SAS », « MICROTITANIUM DIOXIDE MT 500 SAS » ou « MICROTITANIUM DIOXIDE MT 100 SAS » de la société TAYCA,
- de silice, d'alumine, de stéarate d'aluminium et traités par une silicone tels que le produit « STT-30-DS » de la société TITAN KOGYO,
- de silice et traité par une silicone tel que le produit « UV-TITAN X 195 » de la société KEMIRA,
- d'alumine et traités par une silicone tels que les produits « TIPAQUE TTO-55 (S) » de la société ISHIHARA, ou « UV TITAN M 262 » de la société KEMIRA,
- de triéthanolamine tels que le produit « STT-65-S » de la société TITAN KOGYO,
- d'acide stéarique tels que le produit « TIPAQUE TTO-55 (C) » de la société ISHIHARA,
- d'hexamétaphosphate de sodium tels que le produit « MICROTITANIUM DIOXIDE MT 150 W » de la société TAYCA,
- le $TiO_2$ traité par l'octyl triméthyl silane vendu sous la dénomination commerciale « T 805 » par la société DEGUSSA SILICES,
- le $TiO_2$ traité par un polydiméthylsiloxane vendu sous la dénomination commerciale « 70250 Cardre UF TiO2SI3 » par la société CARDRE,
- le $TiO_2$ anatase/rutile traité par un polydiméthylhydrogénosiloxane vendu sous la dénomination commerciale « MICRO TITANIUM DIOXYDE USP GRADE HYDROPHOBIC » par la société COLOR TECHNIQUES.

Les pigments d'oxyde de titane non enrobés sont par exemple vendus par la société TAYCA sous les dénominations commerciales « MICROTITANIUM DIOXIDE MT 500 B » ou « MICROTITANIUM DIOXIDE MT600 B », par la société DEGUSSA sous la dénomination « P 25 », par la société WACKHER sous la dénomination « Oxyde de titane transparent PW », par la société MIYOSHI KASEI sous la dénomination « UFTR », par la société TOMEN sous la dénomination « ITS » et par la société TIOXIDE sous la dénomination « TIOVEIL AQ ».

Les pigments d'oxyde de zinc non enrobés, sont, par exemple, ceux commercialisés sous la dénomination « Z-cote » par la société Sunsmart.

Les pigments d'oxyde de zinc enrobés sont par exemple :

- ceux commercialisés sous la dénomination « Oxide zinc CS-5 » par la société Toshibi (ZnO enrobé par polyméthylhydrogènesiloxane) ;
- ceux commercialisés sous la dénomination « DAITOPERSION ZN-30 » et « DAITOPERSION Zn-50 » par la société Daito (dispersions dans cyclopolyméthylsiloxane /polydiméthylsiloxane oxyéthyléné, contenant 30 % ou 50 % de oxydes de zinc ultrafins enrobés par la silice et le polyméthylhydrogènesiloxane) ;
- ceux commercialisés sous la dénomination « NFD Ultrafine ZnO » par la société Daikin (ZnO enrobé par phosphate

de perfluoroalkyle et copolymère à base de perfluoroalkyléthyle en dispersion dans du cyclopentasiloxane) ;

- ceux commercialisés sous la dénomination « SPD-Z1 » par la société Shin-Etsu (ZnO enrobé par polymère acrylique greffé silicone, dispersé dans cyclodiméthylsiloxane) ;
- ceux commercialisés sous la dénomination « Escalol Z100 » par la société ISP (ZnO traité alumine et dispersé dans le mélange méthoxycinnamate d'éthylhexyle/copolymère PVP-hexadécène/méthicone) ;
- ceux commercialisés sous la dénomination « Fuji ZnO-SMS-10 » par la société Fuji Pigment (ZnO enrobé silice et polyméthylsilsesquioxane).

Les pigments d'oxyde de cérium non enrobés peuvent être par exemple ceux vendus sous la dénomination « COLLOIDAL CERIUM OXIDE » par la société RHONE POULENC.

Les pigments d'oxyde de fer non enrobés sont par exemple vendus par la société MITSUBISHI sous la dénomination « TY-220 ».

Les pigments d'oxyde de fer enrobés sont par exemple vendus par la société BASF sous la dénomination « OXYDE DE FER TRANSPARENT ».

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la *dénomination « SUNVEIL A », ainsi que le mélange de dioxyde de titane et de dioxyde de* zinc enrobé d'alumine, de silice et de silicone tel que le produit « M 261 » vendu par la société KEMIRA ou enrobé d'alumine, de silice et de glycérine tel que le produit « M 211 » vendu par la société KEMIRA.

Selon l'invention, les pigments d'oxyde de titane, enrobés ou non enrobés, sont particulièrement préférés.

Les filtres inorganiques conformes à l'invention peuvent représenter de 0,5 à 40 %, de préférence de 1 à 30 %, du poids total de la composition.

La présente invention concerne, selon un deuxième de ses aspects, des compositions cosmétiques et/ou dermatologiques sous la forme d'une émulsion huile-dans-eau comprenant au moins un ester de l'acide N,N'-diarylméthylène éthylènediaminediacétique et au moins un tensioactif particulier.

Les compositions selon ce deuxième aspect de l'invention sont en particulier destinées au soin et/ou au maquillage des matières kératiniques, et notamment de la peau.

Comme indiqué précédemment, les esters de l'acide N,N'-diarylméthylène éthylènediaminediacétique présentent la particularité d'être solides à température ambiante et de n'être que peu ou pas solubles dans les matières premières liquides utilisées de façon usuelle en cosmétique et/ou en dermatologie.

C'est pourquoi, afin de tirer au mieux profit de leur activité, il est généralement nécessaire de formuler ces composés avec un solvant particulier dédié spécifiquement à leur solubilisation.

Par ailleurs, les formulations sous forme d'émulsions huile-dans-eau sont avantageuses dans le domaine de la cosmétique et de la dermatologie en ce qu'elles présentent généralement une très bonne tolérance sur la peau, et des propriétés sensorielles et/ou organoleptiques satisfaisantes pour le consommateur (notamment en terme de toucher).

Or les inventeurs ont constaté que la réalisation d'émulsions huile-dans-eau avec des tensioactifs anioniques comme les acides gras tel que l'acide stéarique engendrent une dégradation chimique des composés esters induisant une diminution de la quantité de ces composés esters présents dans l'émulsion et donc une perte d'efficacité du produit cosmétique.

Pour ces raisons, il demeure un besoin de disposer de compositions convenant à la formulation d'esters de l'acide N,N'-diarylméthylène éthylènediaminediacétique sous la forme d'émulsions huile-dans-eau plus performantes, ne nuisant pas à la stabilité chimique dudit ester, et ce tout en conservant une bonne tolérance cutanée.

Selon son deuxième aspect, la présente invention a précisément pour objet de proposer une nouvelle formulation galénique d'esters de l'acide N,N'-diarylméthylène éthylènediaminediacétique permettant de s'affranchir des inconvénients précités et de maintenir la stabilité chimique de ces composés de façon durable dans le temps.

Les inventeurs ont en effet découvert, de façon inattendue, que l'association de certains esters de l'acide N, N'-diarylméthylène éthylènediaminediacétique avec certains tensioactifs particuliers permet de donner satisfaction en ces termes, et notamment de conserver la stabilité chimique desdits esters formulés sous la forme d'une émulsion huile-dans-eau.

Dans le cadre de ce deuxième aspect de l'invention, l'invention concerne ainsi, selon un de ses aspects, une composition cosmétique et/ou dermatologique sous la forme d'une émulsion huile-dans-eau comprenant :

(a) au moins un composé de formule générale (I) :

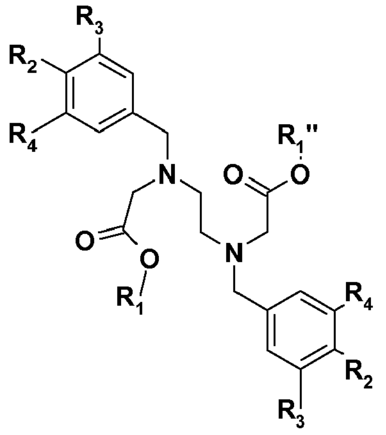

(I)

dans laquelle :

- chaque groupement R1 et R"1 représente indépendamment un atome d'hydrogène ou un radical alkyle saturé linéaire comprenant de 1 à 6 atomes de carbone ou un radical alkyle saturé ramifié comprenant de 3 à 6 atomes de carbone, sous réserve que les deux groupements R1 et R"1 ne représentent pas simultanément un atome d'hydrogène,
- R2, R3 et R4 représentent, indépendamment les uns des autres, un atome d'hydrogène ou un radical -OR5, et
- R5 représente un atome d'hydrogène ou un radical alkyle saturé linéaire comprenant de 1 à 5 atomes de carbone ou un radical alkyle saturé ramifié comprenant de 3 à 5 atomes de carbone,

ou leurs sels,
(b) une quantité efficace d'au moins un solvant dudit composé de formule générale (I), et
(c) au moins un tensioactif ionique choisi parmi les sels alcalins des acides acyl (C10-C22) glutamique, les sels alcalins de palmitoyl sarcosinate, et leurs mélanges.

Le composé de formule générale (I) est avantageusement présent, dans les compositions conformes à ce deuxième aspect de l'invention, sous une forme solubilisée grâce à la présence d'au moins un de ses solvants en une quantité efficace. Sa stabilité chimique est, quant à elle, préservée par la présence d'au moins un tensioactif ionique particulier tel que défini précédemment.

L'association, dans une émulsion huile-dans-eau, d'un ester de l'acide N,N'-diarylméthylène éthylènediaminediacétique conforme à l'invention avec un tel tensioactif ionique permet d'obtenir des compositions cosmétiques et/ou dermatologiques remarquablement efficaces pour les applications mentionnées précédemment.

Les compositions conformes à ce deuxième aspect de l'invention peuvent notamment être destinées à traiter et/ou protéger les matières kératiniques d'êtres humains, en particulier la peau, contre le vieillissement engendré notamment par l'exposition au soleil (rayons ultraviolets).

Ainsi, selon un autre de ses aspects, la présente invention concerne un procédé de traitement non thérapeutique de soin et/ou de maquillage des matières kératiniques, notamment de la peau, comprenant au moins l'étape d'appliquer sur lesdites matières kératiniques, et notamment sur la peau, au moins une composition telle que définie précédemment.

Les esters de l'acide N,N'-diarylméthylène éthylènediaminediacétique considérés selon ce deuxième aspect de l'invention sont des composés de formule générale (I), (Ia) ou (Ib) tels que définis précédemment pour le premier aspect de l'invention.

La quantité d'esters de l'acide N,N'-diarylméthylène éthylènediaminediacétique à mettre en oeuvre dans une composition selon ce deuxième aspect de l'invention dépend de l'effet cosmétique recherché et peut donc varier dans une large mesure.

L'homme de l'art peut aisément, sur la base de ses connaissances générales, déterminer les quantités appropriées.

Les compositions conformes à ce deuxième aspect de l'invention peuvent comprendre de 0,2 à 10 % en poids, de préférence de 0,5 à 5 % en poids, notamment de 0,8 à 3 % en poids, et par exemple au moins 1 % en poids de composé de formule générale (I), (Ia) ou (Ib), en particulier de formule (Ia), par rapport au poids total de ladite composition.

## SOLVANT DE L'ESTER DE L'ACIDE N.N'-DIARYLMETHYLENE ETHYLENEDIAMINEDIACETIQUE

Les compositions conformes à ce deuxième aspect de l'invention comprennent au moins un ester de l'acide N,N'-diarylméthylène éthylènediaminediacétique de formule (I), (Ia) ou (Ib) en association avec au moins une quantité efficace d'un de ses solvants.

A titre de solvant convenable, on peut notamment citer l'éthanol, l'isoparaffine hydrogénée, le squalane, le palmitate d'isopropyle, l'octyl-2-dodécanol, le salicylate de 2-éthyl hexyle, le dipropylène glycol, le myristate d'isopropyle, l'hexylèneglycol, la phényltriméthicone, l'huile d'abricot, l'isononanoate d'isononyle, le diméthyl isosorbide, les esters d'acide aminé de formule (II) :

$$R'1(CO)N(R'2)CH(R'3)(CH2)n(CO)OR'4 \qquad (II)$$

dans laquelle :

- n est un entier égal à 0, 1 ou 2,
- R'1 représente un radical alkyle ou alcényle en C5 à C21, linéaire ou ramifié,
- R'2 représente un atome d'hydrogène ou un groupe alkyle en C1 à C3,
- R'3 représente un radical choisi dans le groupe formé par un atome d'hydrogène, un groupe méthyle, un groupe éthyle, un radical alkyle linéaire ou ramifié en C3 ou C4, et
- R'4 représente un radical alkyle en C1 à C10 linéaire ou ramifié, ou un radical alcényle en C2 à C10 linéaire ou ramifié, ou un reste stérol ; et

l'un de leurs mélanges.

Il s'agira de préférence d'un solvant choisi parmi l'isononanoate d'isononyle, le diméthyl isosorbide, les esters d'acide aminé de formule (II) telle que définie ci-dessus, et l'un de leurs mélanges.

Le solvant considéré selon ce deuxième aspect de l'invention peut être notamment un solvant tel que défini précédemment pour le premier aspect de l'invention.

## TENSIOACTIF IONIQUE

Les compositions conformes à ce deuxième aspect de l'invention comprennent en outre au moins un tensioactif ionique choisi parmi les sels alcalins des acides acyl (C10-C22) glutamique, les sels alcalins de palmitoyl sarcosinate, et leurs mélanges.

A titre de sels alcalins, on peut par exemple citer les sels de sodium, les sels de potassium et les sels de lithium, et de préférence les sels de sodium.

Selon un mode de réalisation, les compositions conformes à ce deuxième aspect de l'invention comprennent au moins un sel alcalin des acides acyl (C10-C22) glutamique, de préférence un sel alcalin des acides acyl (C12-C20) glutamique, et par exemple un sel alcalin des acides acyl (C16-C18) glutamique.

Il peut notamment s'agir d'un des sels alcalins de l'acide stéaroyl glutamique, de l'acide lauroyl glutamique, de l'acide acyl C16 glutamique, de l'acide myristoyl glutamique, de l'acide cocoyl glutamique ou de l'acide acyle de suif hydrogéné glutamique.

De préférence, il s'agira d'un tensioactif ionique choisi parmi le stéaroyl glutamate de sodium, le stéaroyl glutamate disodique, le stéaroyl glutamate de potassium, le lauroyl glutamate de sodium, le lauroyl glutamate disodique, le lauroyl glutamate de potassium, le cocoyl glutamate de sodium, l'acyle de suif hydrogéné glutamate de sodium, et leurs mélanges, et de préférence du stéaroyl glutamate de sodium.

A titre illustratif, on peut, par exemple, citer le stéroyl glutamate de sodium commercialisé par la société AJINOMOTO sous la référence AMISOFT HS 11 PF®.

Selon un autre mode de réalisation, les compositions conformes à ce deuxième aspect de l'invention comprennent au moins un sel alcalin de palmitoyl sarcosinate.

De préférence, il s'agira du palmitoyl sarcosinate de sodium.

Pour des raisons évidentes, la quantité de tensioactif ionique conforme à ce deuxième aspect de l'invention est susceptible de varier dans une large mesure en fonction notamment de la nature chimique et/ou de la quantité dudit ester de l'acide N,N'-diarylméthylène éthylènediaminediacétique et de la nature du tensioactif ionique utilisé. L'ajustement de la quantité de tensioactif ionique conforme à ce deuxième aspect de l'invention fait partie des compétences de l'homme du métier.

A titre illustratif, le tensioactif ionique conforme à ce deuxième aspect de l'invention peut être présent en une quantité en matière active allant de 0,01 à 20 % en poids, plus préférentiellement de 0,1 à 10 % en poids, encore plus préférentiellement de 0,2 à 5 % en poids et plus particulièrement encore de 0,25 à 2 % en poids par rapport au poids

total de la composition.

**EMULSION**

Les compositions conformes à ce deuxième aspect de l'invention se présentent sous la forme d'une émulsion huile-dans-eau.

Outre un tensioactif ionique conforme à ce deuxième aspect de l'invention, ces émulsions peuvent contenir au moins un émulsionnant additionnel choisi parmi les émulsionnants amphotères, anioniques, cationiques ou non ioniques, utilisés seuls ou en mélange. Les émulsionnants additionnels sont bien évidemment choisis de manière appropriée à l'obtention d'une émulsion huile-dans-eau.

A titre d'émulsionnants additionnels utilisables, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; et leurs mélanges tels que le mélange de mono-stéarate de glycéryle et de stéarate de polyéthylène glycol (100 OE) commercialisé sous la dénomination SIMULSOL 165 par la société SEPPIC ; les esters d'acides gras et de sorbitan oxyalkylénés comprenant par exemple de 20 à 100 OE, et par exemple ceux commercialisés sous les dénominations commerciales Tween 20 ou Tween 60 par la société Ubiqema ; les éthers d'alcools gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges comme par exemple le mélange de stéarate de glycéryle et de stéarate de PEG-100, commercialisé sous la dénomination Arlacel 165 par la société Uniqema, ou le mélange de stéarate de glycéryle et de stéarate de PEG-40.

On peut aussi préparer des émulsions sans tensioactifs émulsionnants additionnels ou en contenant moins de 0,5 % du poids total de la composition, en utilisant des composés appropriés, par exemple les polymères ayant des propriétés émulsionnantes tels que les polymères acide acrylique/méthacrylate de stéaryle tels que ceux commercialisés sous les dénominations Carbopol 1342 et Pemulen par la société Noveon ; ou les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ou comme le polymère acrylamide/acrylamido 2-méthyl propane sulfonate de sodium tel que celui commercialisé sous la dénomination Sepigel 305 par la société Seppic (nom INCI : Polyacrylamide/C13-C14 isoparaffme/laureth-7), le copolymère (AMPS/méthacrylate d'alcool C12/C14 éthoxylé) (8 moles OE) (80/20) (Aristoflex LNC® de chez CLARIANT) ; les particules de polymères ioniques ou non ioniques, plus particulièrement des particules de polymère anionique comme notamment les polymères d'acide isophtalique ou d'acide sulfoisophtalique, et en particulier les copolymères de phtalate/sulfoisophtalate/glycol (par exemple diéthylèneglycol/phtalate/isophtalate/1,4-cyclohexane-diméthanol (nom INCI : Diglycol/CHDM/I sophtalates/SIP Copolymer) vendus sous les dénominations Eastman AQ polymer (AQ35S, AQ38S, AQSSS, AQ48 Ultra) par la société Eastman Chemical.

La composition selon ce deuxième aspect de l'invention peut comprendre, en outre, au moins un agent photoprotecteur organique et/ou au moins un agent photoprotecteur inorganique actif dans l'UVA et/ou l'UVB (absorbeurs) pouvant être hydrosolubles ou liposolubles ou bien insolubles dans les solvants cosmétiques couramment utilisés, tel que défini précédemment pour le premier aspect de l'invention.

Le deuxième aspect de l'invention est plus particulièrement illustré par les exemples 7 et 8.

La présente invention concerne, selon un troisième de ses aspects, des compositions cosmétiques et/ou dermatologiques comprenant au moins un ester de l'acide N,N'-diarylméthylène éthylènediaminediacétique et au moins un polymère émulsionnant particulier.

Les compositions selon ce troisième aspect de l'invention sont en particulier destinées au soin et/ou au maquillage des matières kératiniques, et notamment de la peau.

Comme indiqué précédemment, les esters de l'acide N,N'-diarylméthylène éthylènediaminediacétique présentent la particularité d'être solides à température ambiante et de n'être que peu ou pas solubles dans les matières premières liquides utilisées de façon usuelle en cosmétique et/ou en dermatologie.

Or, il est nécessaire que ces composés soient formulés sous une forme solubilisée ou dispersée afin de tirer au mieux profit de leur activité et il est également préférable que cet état soit maintenu au cours du temps pour éviter tout phénomène de recristallisation lors du stockage des compositions comportant de tels composés.

Selon un troisième de ses aspects, la présente invention vise pour sa part à formuler des esters de l'acide N, N'-diarylméthylène éthylènediaminediactétique dans la phase huileuse d'une émulsion huile-dans-eau ou eau-dans-huile-dans-eau.

Les inventeurs ont en effet découvert, de façon inattendue, que l'association de certains esters de l'acide N, N'-diarylméthylène éthylènediaminediacétique avec au moins un polymère émulsionnant particulier permet d'introduire ces composés esters dans la phase huileuse de ce type d'émulsion en évitant leur recristallisation, notamment après un stockage de deux mois à la température ambiante (25 °C).

Dans le cadre de ce troisième aspect de l'invention, l'invention concerne ainsi, selon un de ses aspects, une composition cosmétique et/ou dermatologique sous la forme d'une émulsion huile-dans-eau ou d'une émulsion eau-dans-huile-dans-eau contenant, dans sa phase huileuse,

(a) au moins un composé de formule générale (I) :

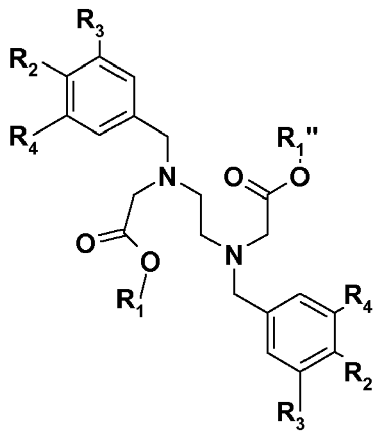

(I)

dans laquelle :

chaque groupement R1 et R"1 représente indépendamment un atome d'hydrogène ou un radical alkyle saturé linéaire comprenant de 1 à 6 atomes de carbone ou un radical alkyle saturé ramifié comprenant de 3 à 6 atomes de carbone, sous réserve que les deux groupements R1 et R"1 ne représentent pas simultanément un atome d'hydrogène,

R2, R3 et R4 représentent, indépendamment les uns des autres, un atome d'hydrogène ou un radical -OR5, et R5 représente un atome d'hydrogène ou un radical alkyle saturé linéaire comprenant de 1 à 5 atomes de carbone ou un radical alkyle saturé ramifié comprenant de 3 à 5 atomes de carbone,

ou leurs sels, et

(b) une quantité efficace d'au moins un polymère amphiphile comportant au moins un motif acide acrylamido 2-méthylpropane sulfonique (AMPS).

Comme il ressort des exemples 9 à 12 ci-après, la présence d'un polymère amphiphile conforme à ce troisième aspect de l'invention permet d'assurer efficacement la présence du composé de formule générale (I) au sein de la phase huileuse de l'émulsion.

La mise en oeuvre à cet effet d'un tel polymère amphiphile permet ainsi avantageusement de s'affranchir de l'utilisation de solvants solubilisants, rendant plus simple la formulation des émulsions contenant les composés esters.

Les compositions conformes à ce troisième aspect de l'invention sont avantageusement mises en oeuvre pour le soin et/ou le maquillage des matières kératiniques, et notamment de la peau.

L'association d'un ester de l'acide N,N'-diarylméthylène éthylènediaminediacétique conforme à l'invention avec un tel polymère amphiphile permet d'obtenir des compositions cosmétiques et/ou dermatologiques remarquablement efficaces pour les applications mentionnées précédemment.

Les compositions conformes à ce troisième aspect de l'invention peuvent notamment être destinées à traiter et/ou protéger les matières kératiniques d'êtres humains, en particulier la peau, contre le vieillissement engendré notamment par l'exposition au soleil (rayons ultraviolets).

Ainsi, selon un autre de ses aspects, la présente invention concerne un procédé de traitement non thérapeutique de soin et/ou de maquillage des matières kératiniques, notamment de la peau, comprenant au moins l'étape d'appliquer sur lesdites matières kératiniques, et notamment sur la peau, au moins une composition telle que définie précédemment.

Les esters de l'acide N,N'-diarylméthylène éthylènediaminediacétique considérés selon ce troisième aspect de l'invention sont des composés de formule générale (I), (Ia) ou (Ib) tels que définis précédemment pour le premier aspect de l'invention.

La quantité d'esters de l'acide N,N'-diarylméthylène éthylènediaminediacétique à mettre en oeuvre dans une composition selon ce troisième aspect de l'invention dépend de l'effet cosmétique recherché et peut donc varier dans une large mesure.

L'homme de l'art peut aisément, sur la base de ses connaissances générales, déterminer les quantités appropriées.

Les compositions conformes à ce troisième aspect de l'invention peuvent notamment comprendre de 0,2 à 10 % en poids, de préférence de 0,5 à 5 % en poids, notamment de 0,8 à 3 % en poids, et par exemple au moins 1 % en poids de composé de formule générale (I), (Ia) ou (Ib), en particulier de formule (Ia), par rapport au poids total de ladite composition.

**POLYMERES EMULSIONNANTS**

Par « polymère émulsionnant » ou « polymère amphiphile », on entend désigner, au sens de l'invention, un polymère possédant des propriétés amphiphiles, c'est-à-dire doté d'au moins une partie hydrophile et d'au moins une partie hydrophobe. Les groupements hydrophiles et les groupements hydrophobes sont bien connus de l'homme du métier.

Au sens de la présente invention, on entend désigner par « polymère » un composé comprenant au moins deux motifs de répétition, en particulier, au moins cinq motifs de répétition.

Les polymères amphiphiles considérés selon ce troisième aspect de l'invention sont des polymères amphiphiles comportant au moins un motif acide acrylamido 2-méthylpropane sulfonique (AMPS).

Les polymères amphiphiles comportant au moins un motif acide acrylamido 2-méthylpropane sulfonique (AMPS) utilisables dans le cadre de ce troisième aspect de la présente invention, également appelés plus simplement « polymères d'AMPS amphiphiles » dans la suite, comportent à la fois une partie hydrophile et une partie hydrophobe comportant au moins une chaîne grasse.

La chaîne grasse présente dans lesdits polymères d'AMPS amphiphiles selon ce troisième aspect de l'invention peut comporter de préférence de 7 à 30 atomes de carbone, et plus préférentiellement de 7 à 22 atomes de carbone.

Les polymères d'AMPS amphiphiles selon ce troisième aspect de l'invention sont notamment choisis parmi les polymères amphiphiles d'au moins un monomère acide acrylamido 2- méthylpropane sulfonique (AMPS) et d'au moins un co-monomère hydrophobe à insaturation éthylénique comportant au moins une partie hydrophobe ayant de 7 à 30 atomes de carbone, en particulier de 7 à 22 atomes de carbone, voire de 12 à 22 atomes de carbone.

Les polymères d'AMPS amphiphiles selon ce troisième aspect de l'invention ont en général un poids moléculaire en poids allant de 50 000 à 10 000 000 g/mol, en particulier de 100 000 à 8 000 000 g/mol et encore plus particulièrement de 100 000 à 7 000 000 g/mol.

Ils peuvent être réticulés ou non réticulés.

Lorsque les polymères d'AMPS amphiphiles selon ce troisième aspect de l'invention sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth) acrylate d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétraallyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Les agents de réticulation peuvent être notamment choisis parmi le méthylène-bis-acrylamide, le méthacrylate d'allyle ou le triméthylol propane triacrylate (TMPTA).

Le taux de réticulation peut varier par exemple de 0,01 à 10 % en moles et de préférence de 0,2 à 2 % en moles par rapport au polymère.

Les polymères d'AMPS amphiphiles selon ce troisième aspect de l'invention peuvent notamment être choisis parmi les polymères amphiphiles statistiques d'AMPS modifiés par réaction avec une n-monoalkylamine ou une di-n-alkylamine en C6-C22 tels que ceux décrits dans la demande de brevet WO 00/31154.

Un polymère amphiphile convenant à ce troisième aspect de l'invention peut comprendre au moins un co-monomère hydrophile à insaturation éthylénique choisi par exemple parmi l'acide acrylique, l'acide méthacrylique ou leurs dérivés alkyl substitués ou leurs esters obtenus avec des mono- ou poly-alkylèneglycols, l'acrylamide, le méthacrylamide, la vinylpyrrolidone, le vinylformamide, l'anhydride maléique, l'acide itaconique ou l'acide maléique ou leurs mélanges.

Un polymère amphiphile selon ce troisième aspect de l'invention peut comprendre au moins un co-monomère hydrophobe à insaturation éthylénique.

Un polymère amphiphile convenant à ce troisième aspect de l'invention peut comprendre au moins une partie hydrophobe choisie parmi les radicaux alkyles linéaires, saturés ou insaturés, comme par exemple le n-octyle, le n-décyle, le n-hexadécyle, le n-dodécyle, l'oléyle, ramifiés comme par exemple l'isostéarique ou cycliques comme par

exemple le cyclododécane ou l'adamantane.

Un polymère d'AMPS amphiphile peut contenir en outre au moins un co-monomère hydrophobe à insaturation éthylènique, comprenant par exemple :

- un radical fluoré ou alkylfluoré en C7-C18 (par exemple le groupement de formule -(CH2)2-(CF2)9-CF3),
- un radical cholestéryle ou un radical dérivé de cholestérol (par exemple l'hexanoate de cholestéryle),
- un groupe polycyclique aromatique comme le naphtalène ou le pyrène,
- un radical siliconé ou alkylsiliconé ou encore alkylfluorosiliconé.

Ces copolymères sont notamment décrits dans le document EP-A-0 750 899, le brevet US-A-5,089,578 et dans les publications de Yotaro Morishima suivantes :

- « Self-assembling amphiphilic polyelectrolytes and their nanostructures », Chinese Journal of Polymer Science Vol. 18, N° 40, (2000), 323-336 ;

- « Micelle formation of random copolymers of sodium 2-(acrylamido)-2- methylpropanesulfonate and a non-ionic surfactant macromonomer in water as studied by fluorescence and dynamic light scattering », Macromolecules 2000, Vol. 33, N° 10 -3694-3704;

- « Solution properties of micelle networks formed by non-ionic moieties covalently bound to an polyelectrolyte: salt effects on rheological behavior », Langmuir, 2000, Vol. 16, N° 12, 5324-5332;

- « Stimuli responsive amphiphilic copolymers of sodium 2-(acrylamido)-2-methylpropanesulfonate and associative macromonomers », Polym. Preprint, Div. Polym. Chem. 1999, 40(2), 220-221.

Ils sont également décrits dans les documents EP 1 069 142, WO 02/44224, WO 02/44225, WO 02/44227, WO 02/44229, WO 02/44230, WO 02/44231, WO 02/44267, WO 02/44268, WO 02/44269, WO 02/44270, WO 02/44271, WO 02/43677, WO 02/43686, WO 02/43687, WO 02/43688, WO 02/43689, au nom de CLARIANT.

Un co-monomère hydrophobe à insaturation éthylénique de l'invention peut être choisi de préférence parmi les acrylates ou les acrylamides de formule (1) suivante :

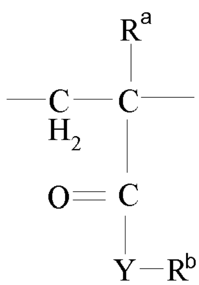

(1)

dans laquelle :

- Ra désigne un atome d'hydrogène, un radical alkyle linéaire ou ramifié en C1-C6, de préférence méthyle ;
- Y désigne O ou NH ;
- Rb désigne un radical hydrophobe comportant une chaîne grasse ayant de 7 à 30 atomes de carbone, et de préférence de 7 à 22, et plus particulièrement de 12 à 22 atomes de carbone.

Le radical hydrophobe Rb est choisi parmi les radicaux alkyles linéaires en C7-C22, saturés ou insaturés (par exemple n-octyle, n-décyle, n-hexadécyle, n-dodécyle, oléyle), ramifiés (par exemple isostéarique) ou cycliques (par exemple cyclododécane ou adamantane) ; les radicaux alkylperfluorés en C7-C18 (par exemple le groupement de formule -(CH2)2-(CF2)9-CF3) ; le radical cholestéryle ou un ester de cholestérol comme l'hexanoate de cholestéryle ; les groupes polycycliques aromatiques comme le naphtalène ou le pyrène.

Parmi ces radicaux, les radicaux alkyles linéaires et ramifiés sont plus particulièrement préférés.

Selon un mode de réalisation préféré de l'invention, le radical hydrophobe Rb peut comporter en plus au moins un motif oxyde d'alkylène et de préférence une chaîne polyoxyalkylénée.

La chaîne polyoxyalkylénée peut être de façon préférentielle constituée de motifs oxyde d'éthylène et/ou de motifs oxyde de propylène et encore plus particulièrement constituée uniquement de motifs oxyde d'éthylène.

Le nombre de moles de motifs oxyalkylénés peut varier en général de 1 à 30 moles et plus préférentiellement de 1 à 25 moles et encore plus préférentiellement de 3 à 20 moles.

Parmi ces polymères, on peut citer :

- les copolymères réticulés ou non réticulés, neutralisés ou non, comportant de 15 à 60 % en poids de motifs AMPS et de 40 à 85 % en poids de motifs (C8-16)alkyl(méth)acrylamide ou de motifs (C8-C16)alkyl(méth)acrylate par rapport au polymère, tels que ceux décrits dans la demande EP-A-0 750 899 ;
- les terpolymères comportant de 10 à 90 % en mole de motifs acrylamide, de 0,1 à 10 % en mole de motifs AMPS et de 5 à 80 % en mole de motifs n-(C6-C 18)alkylacrylamide, par rapport au polymère, tels que ceux décrits dans le brevet US-A-5,089,578 ;
- les copolymères non réticulés d'AMPS partiellement ou totalement neutralisé et de méthacrylate de n-dodécyle, de n-hexadécyle ou de n-octadécyle, tels que ceux décrits dans les articles de Morishima cités ci-dessus ;
- les copolymères réticulés ou non réticulés d'AMPS partiellement ou totalement neutralisé et de n-dodécylméthacrylamide, tels que ceux décrits dans les articles de Morishima cités ci-dessus.

Comme polymères d'AMPS amphiphiles, on peut également citer les copolymères d'AMPS totalement neutralisé et de méthacrylate de n-dodécyle, de n-hexadécyle et/ou de n-octadécyle, ainsi que les copolymères d'AMPS et de n-dodécylméthacrylamide, non réticulés et réticulés.

On citera plus particulièrement les copolymères d'AMPS amphiphiles réticulés ou non réticulés constitués :

(a) de motifs acide 2-acrylamido 2-méthylpropane sulfonique (AMPS) de formule (2) suivante :

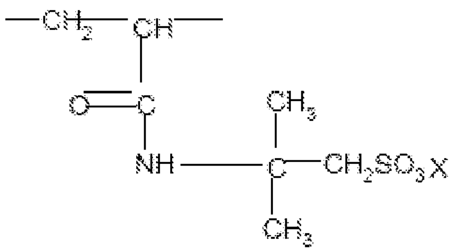

(2)

dans laquelle X est un proton, un cation de métal alcalin, un cation alcalino-terreux ou l'ion ammonium ;

(b) et de motifs de formule (3) suivante :

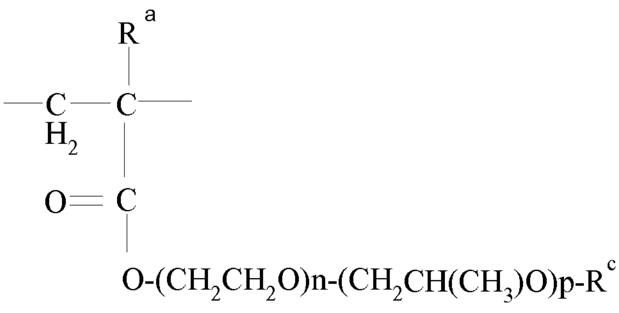

(3)

dans laquelle n et p, indépendamment l'un de l'autre désignent un nombre de moles et varient de 0 à 30, de préférence de 1 à 25 et plus préférentiellement de 3 à 20 sous réserve que n + p soit inférieur ou égal à 30, de préférence inférieur à 25 et encore mieux inférieur à 20 ; Ra désigne un atome d'hydrogène, un radical alkyle linéaire ou ramifié en C1-C6, de préférence méthyle et Rc désigne un alkyle linéaire ou ramifié comportant de 7 à 22 atomes de carbone, de préférence de 12 à 22 atomes de carbone.

Dans la formule (2), le cation X désigne plus particulièrement le sodium ou l'ammonium.

Parmi les monomères de formule (3) on peut citer :

- les esters d'acide (méth)acrylique et d'alcool gras en C10-C18 polyoxethylénés à 8 OE comme le produit GENAPOL C-080® vendu par la Société CLARIANT,
- les esters d'acide (méth)acrylique et d'oxoalcool gras en C11 polyoxyéthyléné à 8 OE comme le produit GENAPOL UD-080® vendu par la Société CLARIANT,
- les esters d'acide (méth)acrylique et d'alcool gras polyoxyéthyléné en C12-C14 à 7 OE comme le produit GENAPOL LA-070® vendu par la Société CLARIANT,
- les esters d'acide (méth)acrylique et d'alcool gras polyoxyéthyléné en C12-C14 à 11 OE comme le produit GENAPOL LA-110® vendu par la Société CLARIANT,
- les esters d'acide (méth)acrylique et d'alcool gras polyoxyéthyléné en C16-C18 à 8 OE comme le produit GENAPOL T-080® vendu par la Société CLARIANT,
- les esters d'acide (méth)acrylique et d'alcool gras polyoxyéthyléné en C16-C18 à 15 OE comme le produit GENAPOL T-150® vendu par la Société CLARIANT,
- les esters d'acide (méth)acrylique et d'alcool gras polyoxyéthyléné en C16-C18 à 11 OE comme le produit GENAPOL T-110® vendu par la Société CLARIANT,
- les esters d'acide (méth)acrylique et d'alcool gras polyoxyéthyléné en C16-C18 à 20 OE comme le produit GENAPOL T-200® vendu par la Société CLARIANT,
- les esters d'acide (méth)acrylique et d'alcool gras polyoxyéthyléné en C16-C18 à 25 OE comme le produit GENAPOL T-250® vendu par la Société CLARIANT,
- les esters d'acide (méth)acrylique et d'alcool gras polyoxyéthyléné en C18-C22 à 25 OE et/ ou d'isoalcool gras polyoxyéthyléné en C16-C18 à 25 OE .

On choisira plus particulièrement :

i. ceux non réticulés pour lesquels p= 0, n = 7 ou 25, Ra désigne un méthyle et Rc représente un mélange d'alkyle en C12-C14 ou en C16-C18,
ii. ceux réticulés pour lesquels p = 0, n = 8 ou 25, Ra désigne un méthyle et Rc représente un mélange d'alkyle en C16-C18.

Ces polymères sont décrits et synthétisés dans la demande EP 1 069 142.

Ces polymères d'AMPS amphiphiles particuliers peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le chlorhydrate de 2,2-azobis-[2-amidinopropane] (ABAH = 2,2-Azo-Bis-[2-Amidinopropane] Hydrochloride), les peroxydes organiques tels que le peroxyde de dilauryle, le peroxyde de benzoyle, l'hydropéroxyde de tert-butyle, des composés peroxydés minéraux tels que le persulfate de potassium ou d'ammonium, ou H2O2 éventuellement en présence de réducteurs.

Ces polymères d'AMPS amphiphiles peuvent être notamment obtenus par polymérisation radicalaire en milieu tert-butanol dans lequel ils précipitent. En utilisant la polymérisation par précipitation dans le tert-butanol, il est possible d'obtenir une distribution de la taille des particules du polymère particulièrement favorable pour ses utilisations.

La réaction peut être conduite à une température comprise entre 0 et 150 °C, de préférence entre 10 et 100 °C, soit à pression atmosphérique, soit sous pression réduite.

Elle peut aussi être réalisée sous atmosphère inerte, et de préférence sous azote.

Les polymères d'AMPS amphiphiles selon ce troisième aspect de l'invention peuvent de préférence être neutralisés partiellement ou totalement par une base minérale telle la soude, la potasse, l'ammoniaque ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils peuvent notamment être totalement ou pratiquement totalement neutralisés, c'est-à-dire neutralisés à au moins 80 %.

La concentration molaire en % des motifs de formule (2) et des motifs de formule (3) dans les polymères d'AMPS amphiphiles selon ce troisième aspect de l'invention peut varier en fonction de l'application cosmétique souhaitée, par exemple de la nature de l'émulsion (huile-dans-eau ou eau-dans-huile) et des propriétés rhéologiques de la formulation recherchées. Elle peut par exemple varier entre 0,1 et 99,9 % en moles.

La distribution des monomères dans les polymères d'AMPS amphiphiles selon ce troisième aspect de l'invention peut être, par exemple, alternée, bloc (y compris multibloc) ou quelconque.

A titre indicatif et sans que cela soit limitatif, on peut citer notamment le copolymère d'AMPS et de méthacrylate d'alcool en C12-C14 éthoxylé (copolymère non réticulé obtenu à partir de Genapol LA-070 et d'AMPS) (nom CTFA : Ammonium Acryloyldiméthyltaurate/Laureth-7 méthacrylate copolymer) commercialisé sous la dénomination ARTISTOFLEX LNC par la société Clariant, le copolymère d'AMPS et de méthacrylate de stéaryle éthoxylé (25 EO) (copolymère réticulé par le triméthylolpropane triacrylate, obtenu à partir de Genapol T-250 et d'AMPS) (nom CTFA : Ammonium Acryloyldimethyltaurate/Steareth-25 Methacrylate Crosspolymer) commercialisé sous la dénomination ARISTOFLEX

HMS par la société Clariant, l'ARISTOFLEX SNC (copolymère AMPS/méthacrylate d'alcool C16/C18 éthoxylés (8 moles EP 80/20 ; nom CTFA : Ammonium acryloyldiméthyltaurate/stéareth-8 méthacrylate copolymer) et l'Aristoflex HMB (copolymère AMPS/méthacrylate de béhényl éthoxyle (25 OE), réticulé par le triméthylolpropane triacrylate (TMPTA).

Dans le cadre de ce troisième aspect de la présente invention, il est entendu par quantité efficace de polymère d'AMPS amphiphile conforme à l'invention une quantité suffisante de ce polymère pour formuler l'ester de l'acide N,N'-diarylméthylène éthylènediaminediacétique de formule (I), (Ia) ou (Ib) dans la phase huileuse de l'émulsion sous une forme durable dans le temps et donc prévenir tout phénomène de recristallisation, notamment au cours du stockage.

Pour des raisons évidentes, la quantité de polymère d'AMPS amphiphile conforme à ce troisième aspect de l'invention suffisante pour formuler l'ester de l'acide N,N' diarylméthylène éthylènediaminediactétique dans la phase huileuse de l'émulsion est susceptible de varier dans une large mesure en fonction notamment de la nature chimique et/ou de la quantité dudit ester de l'acide N,N'-diarylméthylène éthylènediaminediacétique à formuler et de la structure du polymère utilisé. L'ajustement de la quantité de polymère d'AMPS amphiphile conforme à ce troisième aspect de l'invention fait partie des compétences de l'homme du métier.

A titre illustratif, le polymère d'AMPS amphiphile selon ce troisième aspect de l'invention peut être présent en une quantité en matière active allant de 0,05 à 10 % en poids, plus préférentiellement de 0,1 à 5 % en poids, et plus particulièrement encore de 0,5 à 3 % en poids par rapport au poids total de la composition.

Selon un mode de réalisation, le polymère d'AMPS amphiphile conforme à ce troisième aspect de l'invention et le composé de formule générale (I), (Ia) ou (Ib), peuvent être présents en un rapport massique [polymère d'AMPS amphiphile/composé de formule générale (I), (Ia) ou (Ib)] allant de 0,1 à 3, notamment de 0,5 à 2, et par exemple de 0,5 à 1,5, voire égal à 1.

Selon un mode de réalisation, le polymère d'AMPS amphiphile conforme à ce troisième aspect de l'invention et le composé de formule générale (Ia), peuvent être présents en un rapport massique [polymère d'AMPS amphiphile/composé de formule générale (Ia)] allant de 0,1 à 3, notamment de 0,5 à 2, et par exemple de 0,5 à 1,5, voire égal à 1.

## MILIEU PHYSIOLOGIQUEMENT ACCEPTABLE

Les compositions utilisées selon ce troisième aspect de l'invention contiennent un milieu physiologiquement acceptable, c'est-à-dire compatible avec les tissus cutanés tels que la peau et le cuir chevelu.

Ce milieu physiologiquement acceptable comprend au moins une phase huileuse et au moins une phase aqueuse.

Comme huiles utilisables dans la phase huileuse de la composition de ce troisième aspect de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations « Miglyol 810 », « 812 » et « 818 » par la société Dynamit Nobel, l'huile de jojoba, l'huile de beurre de karité;
- les esters et les éthers de synthèse, notamment les esters d'acides gras, comme les huiles de formules R1COOR2 et R1OR2 dans laquelle R1 représente le reste d'un acide gras comportant de 8 à 29 atomes de carbone, et R2 représente une chaîne hydrocarbonée, ramifiée ou non, contenant de 3 à 30 atomes de carbone, comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle ; les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées

comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes ;

- leurs mélanges.

On entend par « huile hydrocarbonée » dans la liste des huiles citées ci-dessus, toute huile comportant majoritairement des atomes de carbone et d'hydrogène, et éventuellement des groupements ester, éther, acide carboxylique et/ou alcool.

La phase huileuse de la composition selon ce troisième aspect de l'invention peut également comprendre des corps solides à la température ambiante (25 °C) comme par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, les cires microcristallines, la cérésine ou l'ozokérite, les cires synthétiques comme les cires de polyéthylène, les cires de Fischer-Tropsch.

Ces corps gras peuvent être choisis de manière variée par l'homme du métier afin de préparer une composition ayant les propriétés, par exemple de consistance ou de texture, souhaitées.

La composition selon ce troisième aspect de l'invention comprend au moins une phase aqueuse qui peut comprendre de l'eau et éventuellement un solvant organique physiologiquement acceptable choisi par exemple parmi les alcools inférieurs comportant de 1 à 8 atomes de carbone et en particulier de 1 à 6 atomes de carbone, comme l'éthanol, l'isopropanol, le propanol, le butanol ; les polyéthylène glycols ayant de 6 à 80 unités oxyde d'éthylène et les polyols comme le propylène glycol, l'isoprène glycol, le butylène glycol, la glycérine et le sorbitol.

Comme indiqué précédemment, les compositions selon ce troisième aspect de l'invention se présentent sous la forme d'une émulsion huile-dans-eau (H/E) ou d'une émulsion triple eau-dans-huile-dans-eau (E/H/E). Ces compositions sont préparées selon les méthodes usuelles.

En outre, les compositions selon ce troisième aspect de l'invention peuvent être plus ou moins fluides et avoir l'aspect d'une crème blanche ou colorée, d'une pommade, d'un lait, d'une lotion, d'un sérum, d'une pâte ou d'une mousse. Elles peuvent être éventuellement appliquées sur la peau sous forme d'aérosol. Elles peuvent aussi se présenter sous forme solide, et par exemple sous forme de stick.

Selon un mode particulier de réalisation de l'invention, la composition selon ce troisième aspect de l'invention est une émulsion huile-dans-eau (H/E).

La proportion de la phase huileuse de l'émulsion peut aller de 5 à 80 % en poids, et de préférence de 5 à 50 % en poids par rapport au poids total de la composition.

Outre le polymère d'AMPS amphiphile conforme à ce troisième aspect de l'invention, les émulsions peuvent contenir au moins un autre émulsionnant (émulsionnant additionnel) choisi parmi les émulsionnants amphotères, anioniques, cationiques ou non ioniques, utilisés seuls ou en mélange. Les émulsionnants sont choisis de manière appropriée suivant l'émulsion à obtenir (E/H/E ou H/E). L'émulsionnant additionnel est généralement présent dans la composition, en une proportion allant de 0,3 à 30 % en poids, et de préférence de 0,5 à 20 % en poids par rapport au poids total de la composition.

Pour les émulsions E/H/E, on peut citer par exemple comme émulsionnants les diméthicone copolyols tels que le mélange de cyclométhicone et de diméthicone copolyol, vendu sous la dénomination « DC 5225 C » par la société Dow Corning, et les alkyl-diméthicone copolyols tels que le Laurylméthicone copolyol vendu sous la dénomination « Dow Corning 5200 Formulation Aid » par la société Dow Corning et le Cétyl diméthicone copolyol vendu sous la dénomination « Abil EM 90® » par la société Goldschmidt. On peut aussi utiliser comme tensioactif d'émulsions E/H/E, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004 et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

De façon connue, la composition cosmétique et/ou dermatologique de ce troisième aspect de l'invention peut contenir également des adjuvants habituels dans le domaine cosmétique et/ou dermatologique tels que les gélifiants, les polymères filmogènes, les conservateurs, les parfums, les charges, les filtres UV, les bactéricides, les absorbeurs d'odeur, les matières colorantes, les extraits végétaux, les actifs cosmétiques et dermatologiques, et les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

La composition selon ce troisième aspect de l'invention peut comprendre, en outre, au moins un agent photoprotecteur organique et/ou au moins un agent photoprotecteur inorganique actif dans l'UVA et/ou l'UVB (absorbeurs) pouvant être hydrosolubles ou liposolubles ou bien insolubles dans les solvants cosmétiques couramment utilisés, tel que défini précédemment pour le premier aspect de l'invention.

Ce troisième aspect de l'invention est plus particulièrement illustré par les exemples 9 à 12.

La présente invention concerne, selon un quatrième de ses aspects, des compositions cosmétiques et/ou dermatologiques sous la forme d'une émulsion huile-dans-eau se présentant sous la forme d'oléosomes et comprenant au moins un ester de l'acide N,N'-diarylméthylène éthylènediaminediacétique et au moins un système tensioactif particulier.

Les compositions selon ce quatrième aspect de l'invention sont en particulier destinées au soin et/ou au maquillage des matières kératiniques, et notamment de la peau.

Comme indiqué précédemment, les esters de l'acide N,N'-diarylméthylène éthylènediaminediacétique présentent la particularité d'être solides à température ambiante et de n'être que peu ou pas solubles dans les matières premières liquides utilisées de façon usuelle en cosmétique et/ou en dermatologie.

C'est pourquoi, afin de tirer au mieux profit de leur activité, il est généralement nécessaire de formuler ces composés avec un solvant particulier dédié spécifiquement à leur solubilisation.

Par ailleurs, les formulations sous forme d'émulsions huile-dans-eau sont avantageuses dans le domaine de la cosmétique et de la dermatologie en ce qu'elles présentent généralement une très bonne tolérance sur la peau, et des propriétés sensorielles et/ou organoleptiques satisfaisantes pour le consommateur (notamment en terme de toucher).

Or les inventeurs ont constaté que la réalisation d'émulsions huile-dans-eau avec des tensioactifs anioniques comme les acides gras tel que l'acide stéarique engendrent une dégradation chimique des composés esters induisant une diminution de la quantité de ces composés esters présents dans l'émulsion et donc une perte d'efficacité du produit cosmétique.

Pour ces raisons, il demeure un besoin de disposer de compositions convenant à la formulation d'esters de l'acide N,N'-diarylméthylène éthylènediaminediacétique sous la forme d'émulsions huile-dans-eau plus performantes, ne nécessitant pas la présence additionnelle de solvants particuliers dudit ester et ne nuisant pas à la stabilité chimique dudit ester.

Selon un quatrième de ses aspects, la présente invention a précisément pour objet de proposer une nouvelle formulation galénique d'esters de l'acide N,N'-diarylméthylène éthylènediaminediacétique permettant de s'affranchir des inconvénients précités et de maintenir notamment la stabilité chimique de ces composés de façon durable dans le temps.

Les inventeurs ont en effet découvert, de façon inattendue, que l'association dans une émulsion huile-dans-eau de certains esters de l'acide N,N'-diarylméthylène éthylènediaminediacétique avec un système tensioactif particulier permet de donner satisfaction en ces termes.

Une telle formulation permet notamment de conserver la stabilité chimique dudit ester présent dans l'émulsion huile-dans-eau.

Elle permet, en outre, de s'affranchir de l'utilisation de solvants particuliers de ces composés esters.

Dans le cadre de ce quatrième aspect de l'invention, l'invention concerne ainsi, selon un de ses aspects, une composition cosmétique et/ou dermatologique sous la forme d'une émulsion huile-dans-eau formée de globules huileux ayant un diamètre moyen inférieur à 0,8 micronmètres et dispersés dans une phase aqueuse, **caractérisée en ce que** chaque globule huileux :

- comprend au moins un composé de formule générale (I) :

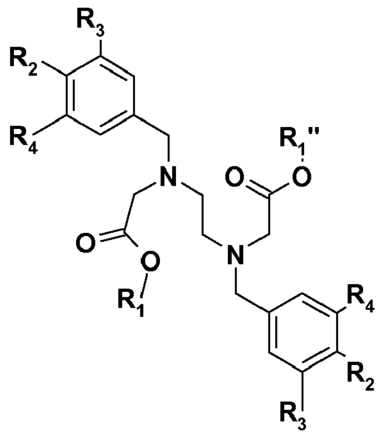

(I)

dans laquelle :

- chaque groupement R1 et R"1 représente indépendamment un atome d'hydrogène ou un radical alkyle saturé linéaire comprenant de 1 à 6 atomes de carbone ou un radical alkyle saturé ramifié comprenant de 3 à 6 atomes de carbone, sous réserve que les deux groupements R1 et R"1 ne représentent pas simultanément un atome d'hydrogène,
- R2, R3 et R4 représentent, indépendamment les uns des autres, un atome d'hydrogène ou un radical -OR5, et
- R5 représente un atome d'hydrogène ou un radical alkyle saturé linéaire comprenant de 1 à 5 atomes de carbone ou un radical alkyle saturé ramifié comprenant de 3 à 5 atomes de carbone,
- ou leurs sels, et

- est unitairement enrobé d'une couche cristal liquide monolamellaire ou oligolamellaire obtenue à partir d'au moins un agent tensioactif non ionique lipophile, d'au moins un agent tensioactif non ionique hydrophile et d'au moins un tensioactif ionique choisi parmi les sels alcalins des acides acyl (C10-C22) glutamique, les sels alcalins de cétylphosphate, les sels alcalins de palmitoyl sarcosinate, et leurs mélanges.

La stabilité chimique du composé (I) est préservée notamment par la présence d'au moins un tensioactif ionique particulier tel que défini précédemment.

L'association, dans une émulsion huile-dans-eau se présentant sous la forme d'oléosomes, d'un ester de l'acide N,N'-diarylméthylène éthylènediaminediacétique conforme à l'invention avec un tel système tensioactif permet d'obtenir des compositions cosmétiques et/ou dermatologiques remarquablement efficaces pour les applications mentionnées précédemment. L'ester ainsi formulé ne recristallise pas dans le temps et l'émulsion reste donc bien homogène.

Les compositions conformes à ce quatrième aspect de l'invention peuvent notamment être destinées à traiter et/ou protéger les matières kératiniques d'êtres humains, en particulier la peau, contre le vieillissement engendré notamment par l'exposition au soleil (rayons ultraviolets).

Ainsi, selon un autre de ses aspects, la présente invention concerne un procédé de traitement non thérapeutique de soin et/ou de maquillage des matières kératiniques, notamment de la peau, comprenant au moins l'étape d'appliquer sur lesdites matières kératiniques, et notamment sur la peau, au moins une composition telle que définie précédemment.

Les esters de l'acide N,N'-diarylméthylène éthylènediaminediacétique considérés selon ce quatrième aspect de l'invention sont des composés de formule générale (I), (Ia) ou (Ib) tels que définis précédemment pour le premier aspect de l'invention.

La quantité d'esters de l'acide N,N'-diarylméthylène éthylènediaminediacétique à mettre en oeuvre dans une composition selon ce quatrième aspect de l'invention dépend de l'effet cosmétique recherché et peut donc varier dans une large mesure.

L'homme de l'art peut aisément, sur la base de ses connaissances générales, déterminer les quantités appropriées.

Les compositions conformes à ce quatrième aspect de l'invention peuvent comprendre de 0,2 à 10 % en poids, de préférence de 0,5 à 5 % en poids, notamment de 0,8 à 3 % en poids, et par exemple au moins 1 % en poids de composé de formule générale (I), (Ia) ou (Ib), en particulier de formule (Ia), par rapport au poids total de ladite composition.

**EMULSION HUILE-DANS-EAU SOUS FORME D'OLEOSOMES**

Les compositions conformes à ce quatrième aspect de l'invention se présentent sous la forme d'oléosomes.

Les oléosomes consistent en une émulsion huile-dans-eau formée par des globules huileux pourvus d'un enrobage cristal liquide lamellaire dispersé dans une phase aqueuse.

Dans le cadre de ce quatrième aspect de la présente invention, il s'agit plus particulièrement d'une émulsion du type huile-dans-eau formée de globules huileux pourvus chacun d'un enrobage cristal liquide lamellaire et dispersés dans une phase aqueuse, caractérisée en ce que chaque globule huileux est unitairement enrobé d'une couche monolamellaire ou oligolamellaire obtenue à partir d'au moins un agent tensioactif non ionique lipophile, d'au moins un agent tensioactif non ionique hydrophile et d'au moins un tensioactif ionique tel que défini précédemment, les globules huileux enrobés ayant un diamètre moyen inférieur à 0,8 micronmètres.

La taille moyenne des globules huileux revêtus est inférieure à 0,8 micronmètres, et de préférence à 0,6 micronmètres, et notamment supérieure à 0,150 micronmètres.

La taille moyenne des globules huileux revêtus peut être exprimée en taille moyenne en nombre notamment mesurée par un granulomètre BROOKHAVEN type BI90PLUS® dont le principe de mesure repose sur la diffusion quasi-élastique de la lumière (QELS).

Comme indiqué précédemment, le système tensioactif comprend au moins un tensioactif ionique choisi parmi les sels alcalins des acides acyl (C10-C22) glutamique, les sels alcalins de cétylphosphate, les sels alcalins de palmitoyl sarcosinate, et leurs mélanges.

A titre de sels alcalins, on peut par exemple citer les sels de sodium, les sels de potassium et les sels de lithium, et de préférence les sels de sodium.

Selon un mode de réalisation, les compositions conformes à ce quatrième aspect de l'invention comprennent au moins un sel alcalin des acides acyl (C10-C22) glutamique, de préférence un sel alcalin des acides acyl (C12-C20) glutamique, et par exemple un sel alcalin des acides acyl (C16-C18) glutamique.

Il peut notamment s'agir d'un des sels alcalins de l'acide stéaroyl glutamique, de l'acide lauroyl glutamique, de l'acide acyl C16 glutamique, de l'acide myristoyl glutamique, de l'acide cocoyl glutamique ou de l'acide acyle de suif hydrogéné glutamique.

De préférence, il s'agira d'un tensioactif ionique choisi parmi le stéaroyl glutamate de sodium, le stéaroyl glutamate disodique, le stéaroyl glutamate de potassium, le lauroyl glutamate de sodium, le lauroyl glutamate disodique, le lauroyl glutamate de potassium, le cocoyl glutamate de sodium, l'acyle de suif hydrogéné glutamate de sodium, et leurs mélanges, et de préférence du stéaroyl glutamate de sodium.

A titre illustratif, on peut, par exemple, citer le stéroyl glutamate de sodium commercialisé par la société AJINOMOTO sous la référence AMISOFT HS 11 PF®.

Selon un autre mode de réalisation, les compositions conformes à ce quatrième aspect de l'invention comprennent au moins un sel alcalin de cétylphosphate.

De préférence, il s'agira du cétyl phosphate de potassium.

A titre illustratif, on peut, par exemple, citer le cétyl phosphate de potassium commercialisé par la société DSM Nutritional Products sous la référence AMPHISOL K®.

Selon un autre mode de réalisation, les compositions conformes à ce quatrième aspect de l'invention comprennent au moins un sel alcalin de palmitoyl sarcosinate.

De préférence, il s'agira du palmitoyl sarcosinate de sodium.

Pour des raisons évidentes, la quantité de tensioactif ionique conforme à ce quatrième aspect de l'invention est susceptible de varier dans une large mesure en fonction notamment de la nature du tensioactif ionique utilisé. L'ajustement de la quantité de tensioactif ionique conforme à ce quatrième aspect de l'invention fait partie des compétences de l'homme du métier.

A titre illustratif, le tensioactif ionique conforme à ce quatrième aspect de l'invention peut être présent en une quantité en matière active allant de 0,1 à 20 % en poids, plus préférentiellement de 0,15 à 10 % en poids, encore plus préférentiellement de 0,2 à 5 % en poids et plus particulièrement encore de 0,25 à 2 % en poids par rapport au poids total de la composition.

Selon un mode de réalisation préférentiel de ce quatrième aspect de l'invention, l'agent tensioactif non ionique lipophile et l'agent tensioactif non ionique hydrophile comportent chacun au moins une chaîne grasse saturée ayant de 12 à 22 atomes de carbone environ. Plus préférentiellement encore, cette chaîne grasse contient de 16 à 22 atomes de carbone.

Selon un autre mode de réalisation préférentiel de ce quatrième aspect de l'invention, l'agent tensioactif non ionique lipophile présente un HLB compris entre environ 2 et environ 5. Comme cela est bien connu, on entend par HLB (Hydrophilic-Lipophilic Balance) l'équilibre entre la dimension et la force du groupe hydrophile et la dimension et la force de groupe lipophile de l'agent tensioactif.

Des exemples de tels agents tensioactifs non ioniques lipophiles sont le distéarate de sucrose, le distéarate

de diglycéryle, le tristéarate de sucrose, le tristéarate de tétraglycéryle, le décastéarate de décaglycéryle, le monostéarate de diglycéryle, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthylèné 2 OE (comportant 2 motifs oxyéthylène), le mono et dibéhénate de glycéryle, le tétrastéarate de pentaérythritol.

Selon un autre mode de réalisation préférentiel de ce quatrième aspect de l'invention, l'agent tensioactif non ionique hydrophile présente de préférence un HLB compris entre environ 8 et environ 12.

On peut citer comme exemples de tels tensioactifs non ioniques hydrophiles les composés suivants : le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthylèné 12 OE et le distéarate de méthylglucose polyoxyéthyléné 20 OE.

L'enrobage selon ce quatrième aspect de l'invention des globules huileux demande de préférence l'utilisation d'une quantité totale d'agent tensioactif non ionique hydrophile, d'agent tensioactif non ionique lipophile et de tensioactif ionique conforme à l'invention comprise entre environ 2 % et environ 6 % en poids par rapport au poids total de la composition. Encore plus préférentiellement, cette quantité est comprise entre 3 % et 4 %. Les quantités relatives de tensioactifs lipophile, hydrophile et de tensioactif ionique conforme à l'invention varient de préférence dans les fourchettes respectives suivantes : 35-55 %/25-40 %/15-35 % en poids par rapport à leur poids total.

La phase grasse, c'est à dire les gouttelettes huileuses enrobées, représente de préférence 5 à 50 % en poids par rapport au poids total de la composition. Encore plus préférentiellement, ce pourcentage est compris entre 10 et 40 %. De préférence, le rapport pondéral huile/eau est égal ou inférieur à 1.

Le rapport pondéral des globules huileux aux éléments constitutifs de l'enrobage est de préférence de 2 à 13 ; encore plus préférentiellement ce rapport va de 6 à 8, et notamment est égal à 7 environ.

Selon un mode de réalisation, l'émulsion a un pH allant de 5,5 à 7,5.

Ces compositions peuvent également comprendre, en outre, au moins un actif lipophile cosmétique ou dermatologique additionnel.

Au sens de ce quatrième aspect de la présente invention, on entend par composé actif lipophile le composé actif en soi lorsqu'il est lui-même une huile ou bien, s'il ne l'est pas, le composé actif dissous dans une huile. Les huiles pouvant être utilisées sont les huiles servant classiquement de support dans les compositions cosmétiques comme par exemple les triglycérides d'acides gras à chaînes courtes, les huiles de silicone, etc.

De telles compositions peuvent être, par exemple, obtenues selon le procédé de préparation décrit dans la demande EP 0 705 593.

La composition selon ce quatrième aspect de l'invention peut comprendre, en outre, au moins un agent photoprotecteur organique et/ou au moins un agent photoprotecteur inorganique actif dans l'UVA et/ou l'UVB (absorbeurs) pouvant être hydrosolubles ou liposolubles ou bien insolubles dans les solvants cosmétiques couramment utilisés tel que défini précédemment pour le premier aspect de l'invention.

Ce quatrième aspect de l'invention est plus particulièrement illustré par les exemples 13 et 14.

Les exemples ci-après sont donnés à titre d'illustration de l'invention et ne doivent pas être interprétés comme limitant sa portée.

**<u>Exemple 1</u> : Préparation du sel d'acide trifluoroacétique du monoester isopropylique de l'acide N,N'-bis-(benzyl)éthylènediamine-N-N'-diacétique**

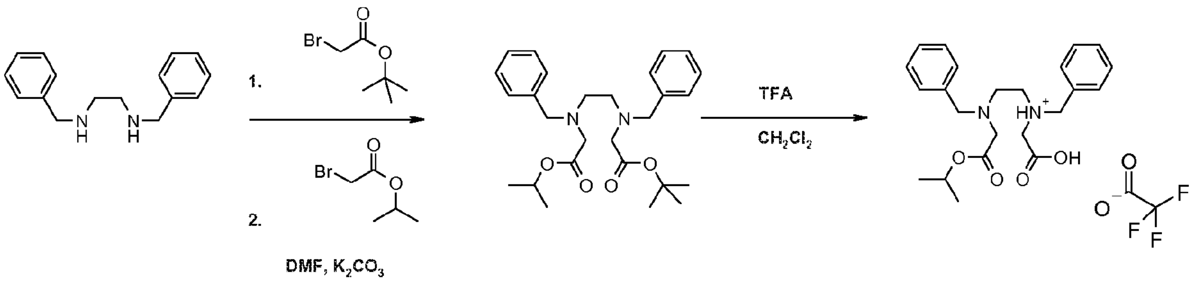


(A)

Dans un réacteur de 2 litres équipé d'une agitation mécanique, on a introduit de la benzathine (A) (36,19 g ;

150 mmol) dans 300 ml de diméthylformamide ainsi que du carbonate de potassium (20,81 g ; 150 mmol). On a maintenu le milieu réactionnel à une température de 0 °C puis on a ajouté au goutte à goutte en 5 heures du tert-butylbromoacétate (27,26 g ; 150 mmol), dilué préalablement dans 500 ml de diméthylformamide. On a agité trente minutes puis on a introduit dans sa totalité de l'isopropylbromocétate (29,37g ; 150 mmol). Le milieu réactionnel a été ramené à température ambiante et l'agitation a été maintenue pendant 17 heures. Après filtration, on a concentré le filtrat issu de la réaction. Le produit obtenu a été repris dans 150 ml de dichlorométhane. On a filtré les sels formés et on a concentré sous pression réduite le filtrat.

On a solubilise cet intermédiaire dans 50 ml de dichlorométhane et on a ajouté une solution d'acide trifluoroacétique à 75 % dans le dichlorométhane. Après 6 heures lorsque la totalité du di-ester mixte a disparu (suivi masse), on a concentré le milieu réactionnel sous pression réduite et on a co-évaporé deux fois avec du toluène.

On a purifié ce produit brut sur fritté de silice avec un système d'éluant dichlorométhane - méthanol. Le pourcentage de méthanol varie de 0 % à 8 %. Dans un premier temps les fractions les plus propres ont été regroupées, séchées. On a obtenu une huile brune collante qui a été reprise dans de l'heptane pendant toute une nuit puis on a filtré le solide obtenu. On a recristallisé ce solide dans un minimum d'eau : on a obtenu une première fraction (lot 1) de 3,1 g d'une poudre blanche.

Dans un second temps, on a regroupé les autres fractions. On a obtenu une huile jaune-orangée que l'on a recristallisée dans de l'éther isopropylique et quelques millilitres d'heptane. On a filtré le précipité pour obtenir une poudre blanche que l'on a lavée dans de l'eau. On a obtenu une seconde fraction (lot 2) de 3 g d'une poudre blanche. Le rendement global est de 10 %. Le produit est obtenu sous la forme de sel d'acide trifluoroacétique (mono-sel).

**Analyses élémentaires**

*Calculé :* C:58.6% ; H : 6.1% ; N : 5.5% ; O : 18.7% ; F : 11.1%
*Mesuré (lot 1)* C : 58.32% ; H : 6.10% ; N:5.39% ; F : 10.94%
*Mesuré (lot* 2*)* : C : 58.42% ; H : 6.12% ; N : 5.45 % ; F : 11.09%

**Exemple 2 : Test de solubilité**

Les essais de solubilité ont été réalisés avec le composé ester diisopropylique de l'acide N,N'-bis (benzyl) éthylènediamine N,N' diacétique.

Dans un bécher, on a versé le solvant testé, on a ajouté une quantité du composé ester sous agitation magnétique, on a laissé reposer entre 1 heure et 24 heures. Puis on a chauffé le mélange à 50 °C puis on a laissé refroidir à la température ambiante (25 °C) pendant 24 heures. On a observé alors si la quantité du composé ester introduite recristallise ou bien reste solubilisée.

On a déterminé de cette façon la quantité de composé ester pouvant être solubilisée dans le solvant testé. La valeur maximale correspond à celle de la quantité à partir de laquelle le composé ester commence à ne pas se solubiliser dans le solvant évalué.

On a obtenu les résultats suivants dans plusieurs solvants évalués :

| Solvant | % en poids de composé ester solubilisé |
|---|---|
| Eau | Insoluble |
| Glycérine | 1 |
| Propylène glycol | 1 |
| Butylène glycol | 1 |
| Cyclopentasiloxane | 4,5 |
| Cyclohexasiloxane | 4,7 |
| Ethanol | 8 |
| Isoparaffine hydrogénée (Parleam de chez NOF) | 9 |
| Squalane | 9 |
| Palmitate d'isopropyle | 9 |

(suite)

| Solvant | % en poids de composé ester solubilisé |
|---|---|
| Eau | Insoluble |
| Octyl-2 dodécanol | 10 |
| Salicylate de 2-éthyl hexyle | 10 |
| Dipropylène glycol | 10,7 |
| Myristate d'isopropyle | 11,5 |
| Hexylèneglyco | 11,5 |
| Phenyltrimethicone (Dow Corning 556) | 12 |
| Huile d'abricot | 12 |
| Isononanoate d'isononyle | 16 |
| N-lauroylsarcosinate d'isopropyle (ELDEW SL-205® de chez AJINOMOTO) | 18 |
| Diméthyl isosorbide | 38 |

On a ainsi constaté que ce composé était mieux solubilisé dans l'isononanoate d'isononyle, le N-lauroylsarcosinate d'isopropyle et le diméthyl isosorbide.

**Exemple 3 : Crème de soin du visage**

Composition $\alpha$ :

*Phase A* :

| | | |
|---|---|---|
| Glycérine | | 5 g |
| Conservateurs (mélange de parabènes - PHENONIP de chez Clariant) | | 1 g |
| Eau | qsp | 100 g |

*Phase B1* :

| | |
|---|---|
| Tristéarate de sorbitan (SPAN 65 V de chez Croda) | 0,9 g |
| Mélange mono/distéarate (36/64) de glycéryle/stéarate de potassium (Tegin Pellets de chez Goldschmidt) | 3 g |
| Stéarate de polyéthylène glycol (40 OE) | 2 g |
| Alcool cétylique | 4 g |
| Alcool stéarylique | 1,20 g |

*Phase B2* :

| | |
|---|---|
| Tétra hydroxycinnamate de dibutyl pentaerithrityle (Tinogard TT de chez Ciba) | 0,20 g |
| Polyisobutène hydrogéné (Parleam de chez NOF) | 5 g |
| Cyclopentasiloxane | 10 g |
| N-lauroylsarcosinate d'isopropyle | 5 g |
| Ester diisopropylique de l'acide N,N'-bis (benzyl)éthylènediamine-N,N' diacétique | 1 g |

La phase B1 a été chauffée à 75 °C, la phase B2 a été chauffée à 50 °C. On a versé la phase B2 dans la phase B1. La phase A a été chauffée à 65 °C. On a versé la phase (B1+B2) dans la phase A sous agitation et on a émulsionné à 65 °C. L'émulsion obtenue est une crème cireuse dont l'application sur le visage permet de protéger la peau contre le stress oxydant engendré par les rayons ultraviolets.
Une composition comparative (composition β), similaire à la composition α mais ne contenant pas le N-lauroylsarcosinate d'isopropyle (remplacé par 5 g d'eau) a également été préparée selon ce même protocole.
Après conservation des compositions α et β pendant 2 mois à 4 °C, à température ambiante et à 45 °C, on a effectué une observation au microscope à l'aide d'un microscope optique du type Leica DMLB muni d'une caméra CCD-IRIS Sony avec un grossissement en * 10 et en lumière polarisée.
On a constaté que la composition α selon l'invention est homogène tandis que la composition β présente des cristaux dus à la mauvaise solubilisation de l'ester diisopropylique de l'acide N,N'-bis (benzyl)éthylènediamine-N,N' diacétique.
Ainsi, la présence de N-lauroylsarcosinate d'isopropyle permet une bonne solubilisation maintenue dans le temps de l'ester diisopropylique de l'acide N,N'-bis (benzyl)éthylènediamine-N,N' diacétique.

**Exemple 4 : Crème de soin du visage**

*Phase A* :

| | |
|---|---|
| Propylène glycol | 3 g |
| Conservateurs (mélange de parabènes - NIPASTAT de chez Clariant) | 0,3 g |
| Phénoxy-2 éthanol | 0,7 g |
| Eau | 63,7 g |

*Phase B1* :

| | |
|---|---|
| Tristéarate de sorbitan (SPAN 65 V de chez Croda) | 0,9 g |
| Mélange mono/distéarate (36/64) de glycéryle/stéarate de potassium (Tegin Pellets de chez Goldschmidt) | 3 g |
| Stéarate de polyéthylène glycol (40 OE) | 2 g |
| Alcool cétylique | 4 g |
| Acide stéarique | 1,20 g |
| Polyisobutène hydrogéné (Parleam de chez NOF) | 5 g |

*Phase B2* :

| | |
|---|---|
| Cyclopentasiloxane | 10g |

*Phase B3* :

| | |
|---|---|
| Tétra hydroxycinnamate de dibutyl pentaerithrityle (Tinogard TT de chez Ciba) | 0,20 g |
| Diméthylisosorbide | 5 g |
| Ester diisopropylique de l'acide N,N'-bis (benzyl)éthylènediamine-N,N' diacétique | 1 g |

La phase B1 a été chauffée à 75 °C, la phase B2 a été chauffée à 50 °C ainsi que la phase B3. On a versé B2 dans B1 puis B3 a été ajoutée. La phase A a été chauffée à 65 °C. On a versé la phase (B1+B2) dans la phase A sous agitation et on a émulsionné à 65 °C. L'émulsion obtenue est une crème cireuse dont l'application sur le visage permet de protéger la peau contre le stress oxydant engendré par les rayons ultraviolets.
A l'issue des 2 mois de conservation (4 °C, à température ambiante et à 45 °C), il n'y a pas eu de recristallisation constatée de l'ester diisopropylique de l'acide N,N'-bis-(benzyl)éthylènediamine-N-N'-diacétique dans cette composition.

**Exemple 5 : Crème de soin du visage**

| | |
|---|---|
| *Phase A* : | |
| Polymère d'acide acrylique réticulé (Carbopol 931 de chez NOVEON) | 0,3 g |
| Triéthanolamine | 0,3 g |
| Conservateurs (mélange de parabènes - NIPASTAT de chez Clariant) | 0,3 g |
| Eau | 67,9 g |

| | |
|---|---|
| *Phase B1* : | |
| Mélange de stéarate de glycéryle et de PEG-100 stéarate (ARLACEL® 165 FL de chez Uniqema) | 2,5 g |
| Stéarate de polyéthylène glycol (50 OE) | 2,5 g |
| Alcool stéarylique | 1 g |
| Alcool cétylique | 1 g |
| Polyisobutène hydrogéné (Parleam de chez NOF) | 5 g |

| | |
|---|---|
| *Phase B2* : | |
| Cyclopentasiloxane | 12 g |

| | |
|---|---|
| *Phase B3* : | |
| Tétra hydroxycinnamate de dibutyl pentaerithrityle (Tinogard TT de chez Ciba) | 0,20 g |
| Isononanoate d'isononyle | 6 g |
| Ester diisopropylique de l'acide N,N'-bis (benzyl)éthylènediamine-N,N' diacétique | 1 g |

La composition a été préparée de la même façon que la composition de l'exemple 2.

A l'issue des 2 mois de conservation (4 °C, à température ambiante et à 45 °C), il n'y a pas eu de recristallisation constatée de l'ester diisopropylique de l'acide N,N'-bis-(benzyl)éthylènediamine-N-N'-diacétique dans cette composition.

La composition appliquée sur le visage permet de protéger la peau contre le stress oxydant engendré par les rayons ultraviolets.

**Exemple 6 :**

On prépare une composition de protection solaire contenant les ingrédients suivants :

| | | |
|---|---|---|
| Propylène glycol | | 6 g |
| Glycérine | | 6 g |
| Sel pentasodique de l'acide éthylène diamine tétracétique | | 0,1 g |
| Eau | qsp | 100 g |
| 2-cyano 3,3-diphénylacrylate de 2-éthyl hexyle | | 10 g |
| 4-tertiobutyl 4'-methoxy dibenzoyl méthane à 33 % dans l'eau | | 2,5 g |
| Drometrizole trisiloxane (Silatrizole de chez Rhodia) | | 4 g |
| Ethylhexyl triazone (Uvinul T150 de chez BASF) | | 2 g |
| N-lauroylsarcosinate d'isopropyle | | 5 g |
| Tétra hydroxycinnamate de dibutyl pentaerithrityle (Tinogard TT de chez Ciba) | | 0,20 g |
| Ester diisopropylique de l'acide N,N'-bis (benzyl)éthylènediamine-N,N' diacétique | | 1 g |
| Mélange de tocophérols dans huile de soja (50/50) (COVI - OX T 50 Cde chez Cognis) | | 0,2 g |

(suite)

| | |
|---|---|
| Mélange de diméthicone copolyol, de cyclopentasiloxane et d'eau (10/88/2) (DC 5225C de Dow Corning) | 1 g |
| Mélange poly diméthylsiloxane alpha-oméga dihydroxyle / cyclopenta diméthylsiloxane (14.7/85.3) (DOW CORNING 1501 FL de chez Dow Corning) | 2 g |
| 2-éthyl hexyl éther de glycérol | 0,5 g |
| Dioxyde de titane (15 nm) traité stéarate d'aluminium/alumine (MT-100 T V de chez Tayca) | 1 g |
| Copolymère acide acrylique/méthacrylate de stéaryle polymérisé dans un mélange acétate d'éthyle/cyclohexane (PEMULEN TR-1 POLYMER de chez Noveon) | 0,4 g |
| Cyclohexasiloxane | 3 g |
| Triéthanolamine | 0,4 g |
| Ethanol | 6 g |

**Exemple 7 (comparatif)**

On a réalisé les émulsions huile-dans-eau suivantes :

| | | α (témoin) | β |
|---|---|---|---|
| - | Stéarate de glycéryle | 3 | 3 |
| - | Stéarate de polyéthylène glycol (40 OE) | 2 | 2 |
| - | Tristéarate de sorbitan (SPAN 65 V de chez Croda) | 0,90 | 0,90 |
| - | Alcool cétylique | 4 | 4 |
| - | Stéaroyl glutamate de sodium (Amisoft HS 11 PF de chez Ajinomoto) | 0 | 1,2 |
| - | Acide stéarique | 1,2 | 0 |
| - | Polyisobutène hydrogéné (Parléam de chez NOF) | 5 | 5 |
| - | N-lauroylsarcosinate d'isopropyle | 5 | 5 |
| - | Ester diisopropylique de l'acide N,N'-bis(benzyl)éthylènediamine N,N'-diacétique | 1 | 1 |
| - | Tétra hydroxycinnamate de dibutyl pentaerithrityle (Tinogard TT de chez Ciba) | 0,2 | 0,2 |
| - | Cyclopentasiloxane | 10 | 10 |
| - | Propylène glycol | 3 | 3 |
| - | Mélange p-hydroxybenzoates de méthyle, butyle, éthyle, propyle, isobutyle (7/57/22/14) | 0,3 | 0,3 |
| - | Triéthanolamine | 0,3 | 0 |
| - | Eau | qsp 100 | qsp 100 |

On a ensuite mesuré la teneur en ester diisopropylique de l'acide N,N'-bis (benzyl)éthylènediamine N,N'-diacétique après 2 mois de stockage à 4 °C, à la température ambiante et à 45 °C.

La teneur en composé diester est mesurée par chromatographie liquide haute performance en phase inverse en utilisant une colonne WATERS / X-Trerra RP18 (5µm) - 150 nm, et comme éluant un gradient Acétonitrile/Tampon acétate de sodium 0,02 M 60/40 et 95/5.

Les conditions chromatographiques sont les suivantes:

Débit : 1 ml/ml
Volume injecté : 20 UL
Détection : 210 nm
Temps de rétention : environ 6,7 minutes

Les deux solutions étalons utilisées sont les suivantes : 12 μg/ml et 120 μg/ml de l'ester diisopropylique de l'acide N,N'-bis (benzyl)éthylènediamine N,N'-diacétique dans l'acétonitrile.

Préparation de l'échantillon à mesurer (solution essai) :

Une masse m de la composition stockée est dispersée dans un volume V d'acétonitrile de façon à obtenir une concentration en composé diester comprise dans la gamme étalon.
On trace la droite de calibration en portant en ordonnée les valeurs de surfaces obtenues sur les chromatogrammes des solutions étalons et en abscisse les concentrations en composé diester dans les solutions étalons.
On déduit de la droite de calibration la concentration dans la composition stockée en utilisant la formule suivante : C = (S - A)/B,

- C étant la concentration en composé diester dans la solution essai (en mg/ml)
- S étant la surface du pic du composé diester sur le chromatogramme essai
- A étant l'ordonnée à l'origine de la droite de calibration du composé diester, et
- B étant la pente de la droite de calibration du composé diester.

La teneur T (en %) du composé diester dans la composition stockée est déterminée par la formule suivante : T = 100 x (C x V)/m,

- C étant la concentration en composé diester dans la solution essai (en mg/ml),
- V étant le volume final après dispersion de l'échantillon (en ml), et
- m étant la quantité d'échantillon de la composition stockée.

Les résultats sont les suivants :

| Teneur après 2 mois de conservation | α (témoin) | β |
|---|---|---|
| à 4°C | 1,0 % | 1,0 % |
| à 45°C | 0,8% | 0,9% |

La mise en oeuvre d'un tensioactif ionique conforme à ce deuxième aspect de l'invention de type stéaroyl glutamate de sodium permet de maintenir, à haute température, la stabilité chimique de l'ester diisopropylique de l'acide N,N'-bis(benzyl)éthylènediamine N,N'-diacétique dans une émulsion huile-dans-eau.

**<u>Exemple 8 :</u>**

On prépare une composition de protection solaire sous la forme d'une émulsion huile-dans-eau contenant les ingrédients suivants :

| | | |
|---|---|---|
| - | Triéthanolamine | 0,88 g |
| - | Sel disodique de l'acide éthylène diamine tétracétique | 0,1 g |
| - | Tétra hydroxycinnamate de dibutyl pentaerithrityle (Tinogard TT de chez Ciba) | 0,20 g |
| - | N-lauroylsarcosinate d'isopropyle | 8 g |
| - | Ester diisopropylique de l'acide N,N'-bis (benzyl) éthylènediamine-N,N' diacétique | 1 g |
| - | Alcool stéarylique | 1 g |
| - | Conservateurs (mélange de parabènes et de phénoxyéthanol - PHENONIP de chez Clariant) | 1 g |
| - | Conservateurs (mélange de parabènes - NIPASTAT de chez Clariant) | 0,25 g |
| - | Salicylate de 2-éthyl hexyle | 5 g |
| - | 4-tertiobutyl 4'-méthoxy dibenzoyl méthane à 33 % dans l'eau | 3 g |
| - | Acide téréphtalylidène dicampho sulfonique | 5 g |

(suite)

| | | |
|---|---|---|
| - | 2-cyano 3,3-diphénylacrylate de 2-éthyl hexyle | 7 g |
| - | Drométrizole trisiloxane (Silatrizole de chez Rhodia) | 1,5 g |
| - | Copolymère acrylamide/acrylamido 2-méthyl propane sulfonate de sodium en émulsion inverse à 40 % dans isohexadecane/eau (Simulgel 600 de chez Seppic) | 2 g |
| - | Particules de copolymère styrène/acrylate (Sunsphères Powder de chez Rohm and Haas) | 2 g |
| - | Cyclopentasiloxane | 3 g |
| - | Glycérine | 5 g |
| - | Mélange de stéarate de glycéryle et de PEG-100 stéarate (ARLACEL® 165 FL de chez Uniqema) | 2 g |
| - | Stéaroyl glutamate de sodium (Amisoft HS 11 PF de chez Ajinomoto) | 1,3 g |
| - | Eau qsp | 100 g |

**Exemple 9 :** Emulsion huile-dans-eau (H/E) stabilisée avec un polymère d'AMPS amphiphile conforme au troisième aspect de l'invention.

*Phase A :*

| | | |
|---|---|---|
| - | Glycérine | 5 % |
| - | Copolymère (AMPS/méthacrylate d'alcool $C_{12}/C_{14}$ éthoxylé) (8 moles OE) (80/20) (Aristoflex LNC® de chez CLARIANT) | 1 % |
| - | Triéthanolamine | 0,0060 % |
| - | Conservateurs | 1 % |
| - | Ester diisopropylique de l'acide N,N'-bis-(benzyl)éthylènediamine-N-N' diacétique | 1 % |
| - | Eau | qsp 100,00 |

*Phase B :*

| | | |
|---|---|---|
| - | Tétra hydroxycinnamate de dibutyl pentaerithrityle (Tinogard TT de chez Ciba) | 0,20 % |
| - | Isoparaffine hydrogénée (Parleam de chez NOF Corporation) | 7 % |
| - | Cyclopentasiloxane | 8 % |
| - | Monoisostéarate de glycéryle | 0,50 % |

La phase A est portée à 60 °C et agitée à l'aide d'une défloculeuse à 450 tours par minute pendant 1 heure. La phase B est portée à 60 °C afin que les constituants solides soient solubilisés. On verse alors la phase B dans la phase A à environ 60 °C.

L'émulsion obtenue est un lait hydratant dont l'application sur le visage permet de protéger la peau contre le stress oxydant engendré par les rayons ultraviolets.

Après conservation de cette composition pendant 2 mois à 4 °C, à la température ambiante et à 45 °C, on a effectué une observation au microscope à l'aide d'un microscope optique du type Leica DMLB muni d'une caméra CCD-IRIS Sony avec un grossissement en * 10 et en lumière polarisée.

Il n'y a pas eu de recristallisation constatée de l'ester diisopropylique de l'acide N,N'-bis-(benzyl)éthylènediamine-N-N'-diacétique dans cette composition.

Ainsi, la présence d'une quantité efficace d'un polymère d'AMPS conforme au troisième aspect de l'invention permet une bonne introduction maintenue dans le temps de l'ester diisopropylique de l'acide N,N'-bis-(benzyl)éthylènediamine-N-N' diacétique dans la phase huileuse de l'émulsion.

**Exemple 10 (comparatif) :** Emulsion huile-dans-eau (H/E) stabilisée avec un polymère émulsionnant de type polymère réticulé acrylates/acrylate d'alkyle en $C_{10}$-$C_{30}$.

*Phase A :*

| | | |
|---|---|---|
| - | Glycérine | 5 % |

(suite)

| | | |
|---|---|---|
| *Phase A :* | | |
| - | Polymère réticulé acrylates/ acrylate d'alkyle en $C_{10}$-$C_{30}$ (Carbopol® Ultrez 20 Polymer de chez Noveon) | 1 % |
| - | Triéthanolamine | 0,98 % |
| - | Conservateurs | 1 % |
| - | Ester diisopropylique de l'acide N,N'-bis-(benzyl)éthylènediamine-N-N'-diacétique | 1 % |
| - | Eau | qsp 100,00 |

| | | |
|---|---|---|
| *Phase B :* | | |
| - | Tétra hydroxycinnamate de dibutyl pentaerithrityle (Tinogard TT de chez Ciba) | 0,20 % |
| - | Isoparaffine hydrogénée (Parleam de chez NOF Corporation) | 7 % |
| - | Cyclopentasiloxane | 8 % |
| - | Monoisostéarate de glycéryle | 0,50 % |

Cette composition a été obtenue en suivant le même protocole expérimental que celui indiqué pour l'exemple 9.

Au bout de seulement 24 heures, une recristallisation de l'ester diisopropylique de l'acide N,N'-bis-(benzyl) éthylènediamine-N-N'-diacétique est constatée.

**Exemple 11(comparatif) :** Emulsion huile-dans-eau (H/E) stabilisée avec un polymère émulsionnant de type cellulose modifiée hydrophobe.

| | | |
|---|---|---|
| *Phase A :* | | |
| - | Glycérine | 5 % |
| - | Cetyl Hydroxy éthyl cellulose (Natrosol® Plus CS 330 de chez Hercules) | 1 % |
| - | Conservateurs | 1 % |
| - | Ester diisopropylique de l'acide N,N'-bis-(benzyl)éthylènediamine-N-N'-diacétique | 1 % |
| - | Eau | qsp 100,00 |

| | | |
|---|---|---|
| *Phase B :* | | |
| - | Tétra hydroxycinnamate de dibutyl pentaerithrityle (Tinogard TT de chez Ciba) | 0,20 % |
| - | Isoparaffine hydrogénée (Parleam de chez NOF Corporation) | 7 % |
| - | Cyclopentasiloxane | 8 % |
| - | Monoisostéarate de glycéryle | 0,50 % |

Cette composition a été obtenue en suivant le même protocole expérimental que celui indiqué pour l'exemple 9.

Au bout de seulement 24 heures, une recristallisation de l'ester diisopropylique de l'acide N,N'-bis-(benzyl) éthylènediamine-N-N'-diacétique est constatée.

**Exemple 12 :** Emulsion solaire huile-dans-eau (H/E) stabilisée avec un polymère d'AMPS amphiphile conforme au troisième aspect de l'invention

| | | |
|---|---|---|
| *Phase A :* | | |
| - | Glycérine | 5 % |
| - | Copolymère (AMPS/méthacrylate d'alcool $C_{12}$/$C_{14}$ éthoxylé) (8 moles OE) | (80/20) |
| | (Aristoflex LNC® de chez CLARIANT) | 1 % |
| - | Triéthanolamine | 0,006 % |

(suite)

*Phase A :*

- Conservateurs 1%
- Ester diisopropylique de l'acide N,N'-bis-(benzyl)éthylènediamine-N-N'-diacétique 1%
- Eau qsp 100,00

*Phase B :*

- Tétra hydroxycinnamate de dibutyl pentaerithrityle (Tinogard TT de chez Ciba) 0,20 %
- Cyano-3,3-diphénylacrylate de 2-éthyl hexyle 3 %
- Polyisobutyène hydrogéné (Parleam de chez NOF Corporation) 7 %
- Cyclopentasiloxane 8 %
- Monoisostérate de glycéryle 0,50 %

Cette composition a été obtenue en suivant le même protocole expérimental que celui indiqué pour l'exemple 9.

Il n'y a pas eu de recristallisation constatée de l'ester diisopropylique de l'acide N-N'bis-(benzyl) éthylène diamine-N-N'-diacétique dans cette composition.

La composition appliquée sur le visage permet de protéger la peau contre les rayons UV.

**Exemple 13 :** Emulsion huile-dans-eau (H/E) sous forme d'oléosomes

On a réalisé une crème de soin du visage ayant la composition α suivante :

*Phase A :*

- Monostéarate de sorbitane polyoxyethyléné (4 OE) (Tween 61 V de chez Croda) 1,76 g
- Tristéarate de sucrose (RYOTO SUGAR ESTER S 370 de chez Mitsubishi Kagaku Foods) 2,64 g
- Alcool stéarylique 1,21 g
- Cholestérol 0,05 g
- Stéarate d'isocétyle 5 g
- Polyisobutène hydrogéné (Parléam de chez NOF) 7 g
- Cyclopentasiloxane 3 g
- Stéaroyl glutamate de sodium (Amisoft HS 11 PF de chez Ajinomoto) 0,25 g
- Ester diisopropylique de l'acide N,N'-bis(benzyl)éthylènediamine N,N'-diacétique 1 g
- Tétra hydroxycinnamate de dibutyl pentaerithrityle (Tinogard TT de chez Ciba) 0,20 g
- Tocophérol 0,20 g

*Phase B :*

- Sel disodique de l'acide éthylène diamine tétracétique 0,1 g
- Phénoxy-2-éthanol 0,50 g
- Butyl parabène 0,10 g
- Méthyl parabène 0,25 g
- Chlorphénésine 0,25 g
- Eau qsp 100 g

*Phase C :*

- Eau 13 g
- Polymère carboxyvinylique (Synthalen K de chez 3V) 0,40 g

*Phase D :*

- Triéthanolamine 0,40 %

La phase A a été chauffée jusqu'à fusion totale des corps gras (environ 85 °C).

La phase B a été chauffée entre 80 °C et 85 °C. La phase B a été ajoutée à la phase A sous agitation très forte. L'émulsion a été passée ensuite sous homogénéisation haute pression. Les phases C et D ont été ajoutées en final.

L'émulsion obtenue sous forme d'oléosomes a un pH égal à 6,10.

On a préparé une composition β similaire en remplaçant le stéaroyl glutamate de sodium par le cétyl phosphate de potassium (AMPHISOL K® de chez DSM Nutritional Products) (0,25 g) et une autre composition γ (témoin) en remplaçant le stéaroyl glutamate de sodium par l'acide stéarique (1,32 g ; avec diminution correspondante de la quantité d'eau).

On a ensuite mesuré la teneur en ester diisopropylique de l'acide N,N'-bis (benzyl)éthylènediamine N,N'-diacétique après 2 mois de stockage à 4 °C et à 45 °C.

La teneur en composé diester est mesurée par chromatographie liquide haute performance en phase inverse en utilisant une colonne WATERS/X-Trerra RP18 (5 μm)-150 nm, et comme éluant un gradient Acétonitrile/Tampon acétate de sodium 0,02 M 60/40 et 95/5.

Les conditions chromatographiques sont les suivantes:

Débit : 1 ml/ml
Volume injecté : 20 UL
Détection : 210 nm
Temps de rétention : environ 6,7 minutes

Les deux solutions étalons utilisées sont les suivantes : 12 μg/ml et 120 μg/ml de l'ester diisopropylique de l'acide N,N'-bis (benzyl)éthylènediamine N,N'-diacétique dans l'acétonitrile.

Préparation de l'échantillon à mesurer (solution essai) :

Une masse m de la composition stockée est dispersée dans un volume V d'acétonitrile de façon à obtenir une concentration en composé diester comprise dans la gamme étalon.

On trace la droite de calibration en portant en ordonnée les valeurs de surfaces obtenues sur les chromatogrammes des solutions étalons et en abscisse les concentrations en composé diester dans les solutions étalons.

On déduit de la droite de calibration la concentration dans la composition stockée en utilisant la formule suivante :

$$C = (S - A)/B,$$

- C étant la concentration en composé diester dans la solution essai (en mg/ml)
- S étant la surface du pic du composé diester sur le chromatogramme essai
- A étant l'ordonnée à l'origine de la droite de calibration du composé diester, et
- B étant la pente de la droite de calibration du composé diester.

La teneur T (en %) du composé diester dans la composition stockée est déterminée par la formule suivante :

$$T = 100 \text{ x } (C \text{ x } V)/m,$$

- C étant la concentration en composé diester dans la solution essai (en mg/ml),
- V étant le volume final après dispersion de l'échantillon (en ml), et
- m étant la quantité d'échantillon de la composition stockée.

Les résultats sont les suivants :

| Teneur après 2 mois de conservation | α | β | γ (témoin) |
|---|---|---|---|
| à 4°C | 1,0 % | 1,0 % | 1,0 % |
| à 45°C | 1,0 % | 1,0 % | 0,8% |

On constate que la mise en oeuvre notamment d'un tensioactif ionique conforme au quatrième aspect de l'invention de type stéaroyl glutamate de sodium ou cétyl phosphate de potassium permet de maintenir, à haute température, la stabilité chimique de l'ester diisopropylique de l'acide N,N'-bis(benzyl)éthylènediamine N,N'-diacétique dans une émulsion huile-dans-eau se présentant sous forme d'oléosomes.

**Exemple 14 :** Emulsion huile-dans-eau (H/E) sous forme d'oléosomes

On prépare une crème de soin du visage ayant la composition suivante :

*Phase A :*

| | | |
|---|---|---|
| - | Eau | 39,25 g |
| - | Méthyl parabène | 0,25 g |
| - | Glycérol | 5 g |
| - | Phénoxy-2 éthanol | 0,7 g |
| - | Sel disodique de l'acide éthylène diamine tétracétique | 0,2g |
| - | Octane-1,2 diol | 0,4 g |

*Phase B :*

| | | |
|---|---|---|
| - | Monostéarate de sorbitane polyoxyethyléné (4 OE) (Tween 61 V de chez Croda) | 1 g |
| - | Tristéarate de sucrose (RYOTO SUGAR ESTER S 370 de chez Mitsubishi Kagaku Foods) | 2 g |
| - | Stéaroyl glutamate de sodium (Amisoft HS 11 PF de chez Ajinomoto) | 0,75 g |
| - | Butyl parabène | 0,15 g |
| - | Cyclohexasiloxane | 1,7 g |
| - | Sébacate de disisopropyle | 2,7 g |
| - | Isononanoate d'isononyle | 5, 6 g |
| - | Ester diisopropylique de l'acide N,N'-bis(benzyl)éthylènediamine N,N'-diacétique | 1 g |
| - | cyano-3,3-diphénylacrylate de 2-éthyl hexyle | 7 g |
| - | 4-tertiobutyl 4'-methoxy dibenzoyl méthane | 3 g |
| - | Salicylate de 2-éthyl hexyle | 5 g |

*Phase C :*

| | | |
|---|---|---|
| - | Eau | Qsp 100 g |
| - | Polymère carboxyvinylique (Synthalen K de chez 3V) | 0,30 g |
| - | Triéthanolamine | 0,3 g |

*Phase D :*

| | | |
|---|---|---|
| - | Eau | 6 g |
| - | Gomme de xanthane | 0,2 g |

Phase E :

- Amidon de maïs estérifié par anhydride octénylsuccinique, sel d'aluminium (Dry Flo Plus de National Starch) 3 g

*Phase F :*

- PEG-12 diméthicone (Silsoft® 880 de chez General Electric) 0,5 g

On chauffe la phase A vers 60-65 °C et la phase B vers 80 °C. On verse la phase A dans la phase B très rapidement au mixer (inversion de phase) en maintenant à 60-65 °C et on émulsionne pendant 15 minutes. A température ambiante, on verse les phases C, D, E, F successivement.

## Revendications

**1.** Composition cosmétique et/ou dermatologique comprenant, dans un milieu physiologiquement acceptable contenant au moins une huile :

(a) au moins un composé de formule générale (Ia) :

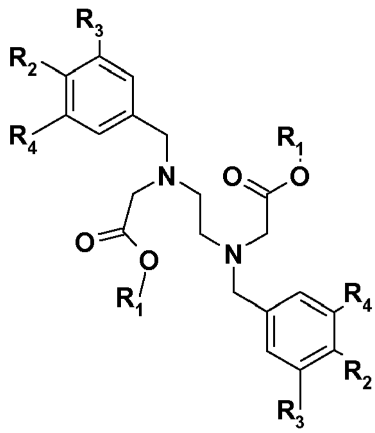

(Ia)

dans laquelle :

- $R_1$ représente un radical alkyle saturé linéaire comprenant de 1 à 6 atomes de carbone ou un radical alkyle saturé ramifié comprenant de 3 à 6 atomes de carbone,
- $R_2$, $R_3$ et $R_4$ représentent, indépendamment les uns des autres, un atome d'hydrogène ou un radical $-OR_5$, et
- $R_5$ représente un atome d'hydrogène ou un radical alkyle saturé linéaire comprenant de 1 à 5 atomes de carbone ou un radical alkyle saturé ramifié comprenant de 3 à 5 atomes de carbone,

ou leurs sels, et

(b) une quantité efficace d'au moins un solvant choisi parmi :

(i) l'isononanoate d'isononyle ;
(ii) le diméthyl isosorbide ;
(iii) les esters d'acide aminé de formule (II) :

$$R'_1(CO)N(R'_2)CH(R'_3)(CH_2)_n(CO)OR'_4 \qquad (II)$$

dans laquelle :

- n est un entier égal à 0, 1 ou 2,
- $R'_1$ représente un radical alkyle ou alcényle en $C_5$ à $C_{21}$, linéaire ou ramifié,
- $R'_2$ représente un atome d'hydrogène ou un groupe alkyle en $C_1$ à $C_3$,
- $R'_3$ représente un radical choisi dans le groupe formé par un atome d'hydrogène, un groupe méthyle, un groupe éthyle, un radical alkyle linéaire ou ramifié en $C_3$ ou $C_4$, et
- $R'_4$ représente un radical alkyle en $C_1$ à $C_{10}$ linéaire ou ramifié, ou un radical alcényle en $C_2$ à $C_{10}$ linéaire ou ramifié, ou un reste stérol ; et

(iv) l'un de leurs mélanges.

**2.** Composition selon la revendication précédente, dans laquelle $R_2$, $R_3$ et $R_4$ représentent un atome d'hydrogène.

**3.** Composition selon l'une quelconque des revendications précédentes, dans laquelle $R_1$ désigne un radical isopropyle.

**4.** Composition selon l'une quelconque des revendications précédentes, dans laquelle le composé de formule générale (Ia) est l'ester diisopropylique de l'acide N, N'-bis-(benzyl)éthylènediamine-N-N'-diacétique.

**5.** Composition selon l'une quelconque des revendications précédentes, comprenant de 0,01 à 5 % en poids, de préférence de 0,1 à 2,5 % en poids, notamment de 0,5 à 1 % en poids de composé de formule générale (Ia) par rapport au poids total de ladite composition.

**6.** Composition selon l'une quelconque des revendications précédentes, dans laquelle ledit ester d'acide aminé est le N-lauroyl sarcosinate d'isopropyle de formule $CH_3$-$(CH_2)_{10}$CO-N($CH_3$)-$CH_2$-COO-CH$(CH_3)_2$.

**7.** Composition selon l'une quelconque des revendications précédentes, comprenant de 0,05 à 25 % en poids, plus préférentiellement de 0,5 à 12,5 % en poids, et plus particulièrement de 2,5 à 5 % en poids, dudit solvant par rapport au poids total de la composition.

**8.** Composition selon l'une quelconque des revendications précédentes, dans laquelle ledit solvant et ledit composé de formule générale (Ia) sont présents en un rapport massique [solvant/composé de formule générale (Ia)] au moins égal à 1,5, notamment allant de 1,5 à 15, et de préférence allant de 1,5 à 10.

**9.** Composition selon l'une quelconque des revendications précédentes, dans laquelle ledit solvant est l'isononanoate d'isononyle et le rapport massique [isononanoate d'isononyle/composé de formule générale (Ia)] est au moins égal 5,25, par exemple allant de 5,25 à 8, et de préférence allant de 5,25 à 6,5.

**10.** Composition selon l'une quelconque des revendications 1 à 8, dans laquelle ledit solvant est le diméthyl isosorbide et le rapport massique [diméthyl isosorbide/composé de formule générale (Ia)] est au moins égal à 1,6, par exemple allant de 1,6 à 10, et de préférence allant de 1,6 à 6.

**11.** Composition selon l'une quelconque des revendications 1 à 8, dans laquelle le solvant est un ester d'acide aminé de formule (II) et le rapport massique [ester d'acide aminé de formule (II)/composé de formule générale (Ia)] est au moins égal à 4,5, par exemple allant de 4,5 à 10, et de préférence allant de 4,5 à 6.

**12.** Composition selon l'une quelconque des revendications précédentes, **caractérisée en ce qu'**elle se présente sous la forme d'une émulsion eau-dans-huile ou huile-dans-eau, de préférence une émulsion huile-dans-eau.

**13.** Composition selon l'une quelconque des revendications précédentes, comprenant en outre au moins un agent photoprotecteur organique et/ou au moins un agent photoprotecteur inorganique actif dans l'UVA et/ou l'UVB.

**14.** Procédé de traitement non thérapeutique de soin et/ou de maquillage des matières kératiniques, notamment de la peau, comprenant au moins l'étape d'appliquer sur lesdites matières kératiniques, et notamment sur la peau, au moins une composition telle que définie en revendications 1 à 13.

Europäisches Patentamt
European Patent Office
Office européen des brevets

# RAPPORT DE RECHERCHE EUROPEENNE

Numéro de la demande
EP 09 17 8776

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 01/87253 A1 (OREAL [FR]; CATROUX PHILIPPE [FR]; COTOVIO JOSE [FR]; DUCHE DANIEL [FR) 22 novembre 2001 (2001-11-22)<br>* page 7, ligne 20,21; revendications 1,5,9,10 *<br>----- | 1-14 | INV.<br>A61K8/44<br>A61Q19/00 |
| A,D | WO 94/11338 A1 (OREAL [FR]; GALEY JEAN BAPTISTE [FR]; DUMATS JACQUELINE [FR]) 26 mai 1994 (1994-05-26)<br>* revendications; exemple 16 *<br>----- | 1-14 | |
| A | FR 2 850 578 A1 (OREAL [FR])<br>6 août 2004 (2004-08-06)<br>* page 10, ligne 28,29; revendications *<br>----- | 1-14 | |

DOMAINES TECHNIQUES RECHERCHES (IPC)

A61K
A61Q

1

Le présent rapport a été établi pour toutes les revendications

| Lieu de la recherche | Date d'achèvement de la recherche | Examinateur |
|---|---|---|
| Berlin | 20 janvier 2010 | Boeker, Ruth |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 09 17 8776

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-01-2010

| Document brevet cité au rapport de recherche | Date de publication | Membre(s) de la famille de brevet(s) | Date de publication |
|---|---|---|---|
| WO 0187253 A1 | 22-11-2001 | AT 277591 T | 15-10-2004 |
| | | AU 6041901 A | 26-11-2001 |
| | | DE 60106014 D1 | 04-11-2004 |
| | | DE 60106014 T2 | 06-10-2005 |
| | | EP 1282394 A1 | 12-02-2003 |
| | | ES 2223849 T3 | 01-03-2005 |
| | | FR 2809002 A1 | 23-11-2001 |
| | | JP 2003533458 T | 11-11-2003 |
| | | US 2004039059 A1 | 26-02-2004 |
| WO 9411338 A1 | 26-05-1994 | AT 185548 T | 15-10-1999 |
| | | CA 2149431 A1 | 26-05-1994 |
| | | DE 69326776 D1 | 18-11-1999 |
| | | DE 69326776 T2 | 02-03-2000 |
| | | EP 0668854 A1 | 30-08-1995 |
| | | ES 2136723 T3 | 01-12-1999 |
| | | JP 3510627 B2 | 29-03-2004 |
| | | JP 8506090 T | 02-07-1996 |
| | | US 5703095 A | 30-12-1997 |
| | | US 5629436 A | 13-05-1997 |
| FR 2850578 A1 | 06-08-2004 | JP 2004307469 A | 04-11-2004 |
| | | US 2004228889 A1 | 18-11-2004 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9411338 A
- EP 1044676 A
- EP 0928608 A
- FR 2796550
- US 5412004 A
- US 5811487 A
- US 5624663 A
- EP 0669323 A
- US 2463264 A
- US 5237071 A
- US 5166355 A
- GB 2303549 A
- DE 19726184
- EP 0893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 1133981 A
- WO 04006878 A
- WO 05058269 A
- WO 06032741 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0518773 A
- WO 0031154 A
- EP 0750899 A
- US 5089578 A
- EP 1069142 A
- WO 0244224 A
- WO 0244225 A
- WO 0244227 A
- WO 0244229 A
- WO 0244230 A
- WO 0244231 A
- WO 0244267 A
- WO 0244268 A
- WO 0244269 A
- WO 0244270 A
- WO 0244271 A
- WO 0243677 A
- WO 0243686 A
- WO 0243687 A
- WO 0243688 A
- WO 0243689 A
- EP 0705593 A


### Littérature non-brevet citée dans la description


- Symetrical Triazine Derivatives. IP.COM Journal. IP.COM INC WEST HENRIETTA, 20 Septembre 2004
- Self-assembling amphiphilic polyelectrolytes and their nanostructures. *Chinese Journal of Polymer Science,* 2000, vol. 18 (40), 323-336
- Micelle formation of random copolymers of sodium 2-(acrylamido)-2- methylpropanesulfonate and a non-ionic surfactant macromonomer in water as studied by fluorescence and dynamic light scattering. *Macromolecules,* 2000, vol. 33 (10), 3694-3704
- Solution properties of micelle networks formed by non-ionic moieties covalently bound to an polyelectrolyte: salt effects on rheological behavior. *Langmuir,* 2000, vol. 16 (12), 5324-5332
- Stimuli responsive amphiphilic copolymers of sodium 2-(acrylamido)-2-methylpropanesulfonate and associative macromonomers. *Polym. Preprint, Div. Polym. Chem.,* 1999, vol. 40 (2), 220-221